# EUROPEAN PATENT SPECIFICATION

(11) **EP 1 431 315 B1**
(45) Date of publication and mention of the grant of the patent: **17.10.2007**
(21) Application number: 03258015.1
(22) Date of filing: 18.12.2003
(51) Int. Cl.: C08F 2/50, C08G 59/00, A61K 6/00

(54) **Photopolymerization initiator and photopolymerizable composition**
Photopolymerisationsinitiator und photopolymerisierbare Zusammensetzung
Initiateur de photopolymérisation et composition photopolymérisable.

(30) Priority: 18.12.2002 JP 2002367080
(43) Date of publication of application: 23.06.2004
(73) Proprietor: TOKUYAMA CORPORATION, Shunan-shi, Yamaguchi 745-0053 (JP); Tokuyama Dental Corporation, Tokyo 110-0016 (JP)
(72) Inventor: Suzuki, Takeshi, Tokyo 110-0016 (JP); Kazama, Hideki, Tokyo 110-0016 (JP)
(74) Representative: Benson, John Everett

(56) References cited:
- EP-A- 0 927 726
- WO-A-02/060973
- GB-A- 2 070 614
- DATABASE WPI Section Ch, Week 199952 Derwent Publications Ltd., London, GB; Class A60, AN 1999-604921 XP002291308 & JP 11 263804 A (NIPPON SODA CO.), 28 September 1999 (1999-09-28)

## Description

### 1. Field of the Invention

The present invention relates to a photopolymerization initiator capable of efficiently polymerizing both a cationically polymerizable monomer and a radically polymerizable monomer, and to a photopolymerizable composition containing the above photopolymerization initiator. More particularly, the invention relates to a photopolymerizable composition that can be favorably used as the dental materials.

### 2. Description of the Related Art

To restore a tooth that is damaged due to caries or breakage, there is usually used a photocurable filling restorative called composite resin owing to its easy use and high aesthetic appearance. The above composite resin usually comprises a polymerizable monomer, a filler and a polymerization initiator..As the polymerizable monomer, there has been used a (meth)acrylate type radically polymerizable monomer from the standpoint of its good photopolymerizable property.

However, the radically polymerizable monomer is impaired for its polymerization due to oxygen. When polymerized and cured in an oral cavity, therefore, an unpolymerized layer or a layer of a low polymerization degree remains on the surface thereof acquiring color or changing color with the passage of time making it difficult to obtain aesthetic appearance to a sufficient degree. By using the conventional dental composite resins using the radically polymerizable monomer, therefore, the surfaces thereof must be sufficiently polished after it is polymerized and cured in the oral cavity so that good aesthetic appearance is fully exhibited.

Besides, the (meth)acrylate type radically polymerizable monomer has a problem of large volumetric shrinkage due to polymerization. Namely, a cavity in the tooth that must be restored is filled with a restorative such as a composite resin which is, then, polymerized and cured by being irradiated with light through the surface of the restorative that is filled. Due to volumetric shrinkage by polymerization, however, there is produced a stress in a direction to float on the interface of the tooth creating a gap between the tooth and the restorative. To cope with the shrinking stress due to polymerization, therefore, there have been proposed a variety of dental adhesives that produce very strong adhering forces. However, the state of tooth varies depending upon the individuals or depending upon the teeth even in the same person. Even by using the above dental adhesives, therefore, a perfect adhesion is not necessarily accomplished for all teeth. Therefore, it has been urged to provide a dental composite resin which undergoes the volumetric shrinkage due to the polymerization as little as possible and which does not develop gap that results from the volumetric shrinkage due to the polymerization. To accomplish a high adhering force, further, the above dental adhesives require complex technique causing an increase in the cost. Therefore, it has further been desired to simplify the steps of adhering operation.

There has also been known cationically polymerizable monomers such as an epoxide as polymerizable monomers that are not impaired for their polymerization by oxygen and that undergo little volumetric shrinkage upon the polymerization. However, the photo-radical polymerization initiators such as an α-diketone and an acylphosphine oxide compound, that are usually used for the dental applications, are not capable of polymerizing the cationically polymerizable monomers. Therefore, a photo-cationic polymerization initiator is necessary.

As the photo-cationic polymerization initiators, there have been known photo acid-generating compounds such as an iodonium salt type compounds and sulfonium salt type compounds. However, these photo acid-generating compounds do not, usually, absorb light in the visible to near ultraviolet regions, and cannot excite the polymerization reaction to a sufficient degree even by using a source of visible light that is used for the dental applications, e.g., even by using a source of light such as a halogen lamp (370 to 550 nm). In order to obtain a sufficient degree of polymerization activity even by the irradiation with light in the visible light region, therefore, there has been proposed to blend a photo acid-generating compound with additives.

For example, there has been proposed to use a photo acid-generating agent in combination with a polycyclic aromatic compound such as an anthracene or an anthracene derivative having an alkoxy group or an acyloxy group (documents 1 to 9).
Document 1: A. Yamaoka, G., Matsunaga, "Photopolymer Technology", Nikkan-Kogyo Shimbunsha Co., pp. 38-46
Document 2: The Society of Polymer Science, Japan, "Synthesis and Reaction of polymer (1), Synthesis of addition polymerized polymer, Kyoritsu Shuppansha Co., pp. 400-404
Document 3: K. Morio, H. Tsuchiya, T. Endo, "Modern Progress in the Photo-Initiated Cationic Polymerization", Functional Material, CMC Co., October, 1985, pp. 5-13
Document 4: Japanese Unexamined Patent Publication (Kokai) No. 8-20728
Document 5: Japanese Unexamined Patent Publication (Kokai) No. 10-147608
Document 6: Japanese Unexamined Patent Publication (Kokai) No. 11-199681
Document 7: Japanese Unexamined Patent Publication (Kokai) No. 2000-7716
Document 8: Japanese Unexamined Patent Publication (Kokai) No.2001-81290
Document 9: Japanese Unexamined Patent Publication (Kokai) No. 11-322952

The photopolymerizable compositions blended with the fused polycyclic aromatic compounds disclosed in the above documents are surely improved for their polymerization property upon the irradiation with visible light as compared to when they are not blended with the above compounds. However, they are polymerized at a small rate and are not still satisfactory as dental polymerization initiators which must be cured within short periods of time in the oral cavity and are not satisfactory, either, from the standpoint of depth of curing.

There has further been proposed to use a photo acid-generating compound in combination with a photo-radical generator such as camphor quinone (documents 10 to 12).
Document 10: International Patent Publication No. 10-508067
Document 11: Japanese Unexamined Patent Publication (Kokai) No. 11-130945
Document 12: International Patent Publication No. 2001-520758)

When the photo-radical generator is used in combination with the photo acid-generating compound as described above, the polymerization is improved without, however, accompanied by a polymerization rate that is high enough for dental applications.

There has further been proposed a cationic polymerization initiator obtained by blending a photo acid-generating compound with an anthracene derivative having an alkoxy group and with a thioxanthone derivative (document 13).
Document 13: Japanese Unexamined Patent Publication (Kokai) No. 11-263804

The polymerizable composition blended with the above cationic polymerization initiator exhibits further improved polymerization property as compared to the polymerizable composition described in the above Documents and features excellent curing rate. However, the depth of curing is shallow and there remains much room for improvement-for using it as the polymerization initiator for curing the dental material that is filled in a deep cavity.

When the dental composite resin is used as described above, further, there is usually used a dental adhesive. However, the dental adhesives that have been placed in the market up to now or that have hitherto been proposed comprise mostly and chiefly the (meth)acrylate type radically polymerizable monomers. Even if it is presumed that the composite resin does not at all produce shrinking stress upon the polymerization, it may often happen that the mechanical holding force is not at all expected depending upon the form of decaying or breakage. In such a case, the dental adhesive must be used. In the oral cavity, further, there are produced chewing stress and thermal stress. To cope with these stresses, it is often desired to use the dental adhesive. Usually, however, the radically polymerizable monomer and the cationically polymerizable monomer do not copolymerize with each other. Therefore, the interfaces of adhesion are not fully bonded (adhered) together between the composite resin comprising the cationically polymerizable monomer and the adhesive comprising chiefly the radically polymerizable monomer. As a result, a gap forms therebetween even by the slightest stress and, depending upon the cases, the restorative (cured composite resin) is split off. To solve this problem, it can be contrived to further blend the composite resin with a radically polymerizable monomer in addition to the cationically polymerizable monomer. As described above, however, the photo-cationic polymerization initiators are not quite capable of polymerizing the radically polymerizable monomers or are very little capable of polymerizing the radically polymerizable monomers. In order to polymerize the radically polymerizable monomers, therefore, it is necessary to blend the radical polymerization initiator. Use of the cationic polymerization initiator and the radical polymerization initiator in combination, however, requires cumbersome control work at the time of production, and is not desirable.

### SUMMARY OF THE INVENTION

It is, therefore, an object of the present invention to provide a photopolymerization initiator which makes it possible to obtain a photopolymerizable composition that can be cured sufficiently deeply and at a sufficiently high rate by the irradiation with visible light that is widely used in the field of dental applications, and is particularly suited for the dental applications.

Another object of the present invention is to provide a photopolymerization initiator capable of efficiently polymerizing not only the cationically polymerizable monomers but also the radically polymerizable monomers.

A further object of the present invention is to provide a photopolymerizable composition containing the above photo-cationic polymerization initiator.

The present inventors have conducted keen study in an effort to solve the above-mentioned problems, and have discovered the fact that a combination of a photo acid-generating compound, a particular photo radical-generating compound and a particular fused polycyclic aromatic compound, is useful as a novel photo-cationic polymerization initiator for achieving the above-mentioned objects. The inventors have further forwarded the study, discovered that the above,polymerization initiator is capable of efficiently polymerizing even the radically polymerizable monomers, and have finished the present invention.

According to the present invention, there is provided a photopolymerization initiator comprising (A) a photo acid-generating compound, (B) a photo oxidation radical-generating compound, and (C) a fused polycyclic aromatic compound having a fused aromatic ring and a molecular structure in which at least one carbon atom adjacent to a ring-fusion carbon atom in the fused aromatic ring is bonded to a saturated carbon atom having at least one hydrogen atom.

According to the present invention, further, there is provided a photopolymerizable composition containing the above photopolymerization initiator.

### DETAILED DESCRIPTION OF THE INVENTION

### (A) Photo acid-generating compounds.

The photo acid-generating compound (A) used for the photocationic polymerization initiator of the present invention directly generates a Bronsted acid or a Lewis acid upon the irradiation with ultraviolet rays, and there can be used any known compound without limitation.

A variety of photo acid-generating compounds have been described in the above-mentioned prior technical literatures. Concrete examples include a diaryliodonium salt compound, a sulfonium salt compound, a sulfonic acid ester compound, and a halomethyl-substituted-S-triazine derivative.

Among the above-mentioned photo acid-generating compounds, the diaryliodonium salt is best suited for the present invention on account of its particularly high polymerization activity.

A representative diaryliodonium salt compound is expressed by the following general formula (2), 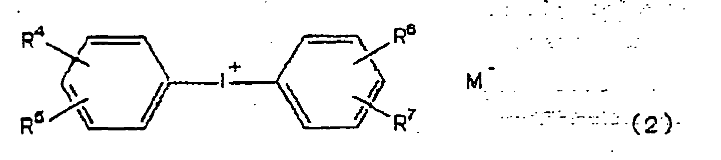 wherein R⁴, R⁵, R⁶ and R⁷ are, independently from each other, hydrogen atoms, halogen atoms, alkyl groups, aryl groups, alkenyl groups, alkoxy groups, aryloxy groups or nitro groups, M⁻ is a halide ion, p-toluene sulfonato ion, perfluoroalkyl sulfonato ion, tetrafluoroborate ion, tetrakis(pentafluorophenyl) borate ion, tetrakis(pentafluorophenyl) garlate ion, hexafluorophosphate ion, hexafluoroarsenato ion or hexafluoroantimonate ion.

Concrete examples of the diaryliodonium salt represented by the above general formula (1) include chloride, bromide, p-toluene sulfonato, perfluoroalkyl sulfonate (e.g., trifluoromethane sulfonato), tetrafluoroborate, tetrakis(pentafluorophenyl) borate, tetrakis(pentafluorophenyl) garlate, hexafluorophosphate, hexafluoroarsenato and hexafluoroantimonate of the following diaryliodonium. Examples of diaryliodonium forming salts:

Diphenyl iodonium, bis(p-chlorophenyl) iodonium, ditolyl iodonium, bis(p-tert-butylphenyl) iodonium, p-isopropylphenyl-p-methylphenyl iodonium, bis(m-nitrophenyl) iodonium, p-tert-butylphenylphenyl iodonium, p-methoxyphenylphenyl iodonium, bis(p-methoxyphenyl) iodonium, p-octyloxyphenylphenyl iodonium, and p-phenoxyphenylphenyl iodonium.

Among the above diaryl iodonium salts according to the present invention, it is desired to use p-toluene sulfonato, perfluoroalkyl sulfonato (e.g., trifluoromethane sulfonato), tetrafluoroborate, tetrakispentafluorophenyl borate, tetrakispentafluorophenyl garlate, hexafluorophosphate, hexafluoroarsenato, and hexafluoroantimonate. Among them, it is most desired to use hexafluoroantimonate, tetrakispentafluorophenyl borate, and tetrakispentafluorophenyl garlate on account of their low nucleophilic properties.

In the present invention, further, examples of the sulfonium salt compound preferably used as the photo acid-generating compound in addition to the above-mentioned diaryl iodonium salts, include salts of sulfonium, such as dimethylphenacyl sulfonium, dimethylbenzyl sulfonium, dimethyl-4-hydroxyphenyl sulfonium, dimethyl-4-hydroxynaphthyl sulfonium, dimethyl-4,7-dihydroxynaphthyl sulfonium, dimethyl-4,8-dihydroxynaphthyl sulfonium, triphenyl sulfonium, p-tolyldiphenyl sulfonium, p-tert-butylphenyldiphenyl sulfonium and diphenyl-4-phenylthiophenyl sulfonium, which may be chloride, bromide, p-toluene sulfonato, trifluoromethane sulfonato, tetrafluoroborate, tetrakispentafluorophenyl borate, tetrakispentafluorophenyl garlate, hexafluorophosphate, hexafluoroarsenato and hexafluoroantimonate.

Concrete examples of the sulfonic acid ester compound include benzoin tosylate, α-methylolbenzoin tosylate, o-nitrobenzyl p-toluene sulfonato, and p-nitrobenzyl 9,10-diethoxyanthracene-2-sulfonato. Concrete examples of the halomethyl substituted-S-triazine derivative include 2,4,6-tris(trichloromethyl) S-triazine, 2-methyl-4,6-bis(trichloromethyl)-S-triazine, 2-phenyl-4,6-bis(trichloromethyl)-S-triazine, and 2-methyl-4,6-bis(tribromomethyl)-S-triazine.

In the present invention, the above photo acid-generating compounds may be used in one kind or being mixed together in two or more kinds.

There is no particular limitation on the amount of using the photo acid-generating compound provided it is used in an amount enough for initiating the polymerization by the irradiation with light. In order to conduct the polymerization at a suitable rate while obtaining various properties (e.g., weatherability and hardness) of the cured material, however, it is desired that the photo acid-generating compound is used in an amount of 0.001 to 10 parts by mass and, more preferably, 0.01 to 5 parts by mass per 100 parts by mass of the polymerizable monomer that will be described later.

### (B) Photo oxidation radical-generating compounds.

A photo oxidation radical-generating compound which is the component (B) contained in the photopolymerization initiator of the present invention generates an active radical species by its own action like an oxidizing agent (which is reduced) upon the irradiation with light. There can be used, for example, a hydrogen-abstracting radical-generating compound that forms a radical by abstracting a hydrogen atom from a hydrogen donor upon being excited by light, a self-cleaving radical-generating compound which undergoes the self cleavage upon being excited by light to generate a radical which, then, abstracts a hydrogen atom from the hydrogen donor, and an electron-abstracting radical-generating compound which generates a radical by abstracting an electron directly from the electron donor upon being excited by light, without any particular limitation.

On the other hand, when there is used a compound of the type that donates electron to other compounds after the self-cleavage (e.g., benzyldimethyl ketal) or a compound that directly turns into a radical upon losing electron by being excited by light (e.g., anthracene, phenothiazine, etc.), however, the polymerizing activity is not improved and the effect of the present invention is not obtained unlike the case of using the photo oxidation radical-generating compound.

In the present invention, examples of the hydrogen-abstracting radical-generating compound include diaryl ketone compound, α-diketone compound and ketocoumarin compound.

As the diaryl ketone compound, there can be exemplified 4,4-bis(dimethylamino)benzophenone, 9-fluorenone, 3,4-benzo-9-fluorenone, 2-dimethylamino-9-fluorenone, 2-methoxy-9-fluorenone, 2-chloro-9-fluorenone, 2,7-dichloro-9-fluorenone, 2-bromo-9-fluorenone, 2,7-dibromo-9-fluorenone, 2-nitro-9-fluorenone, 2-acetoxy-9-fluorenone, benzanthrone, anthraquinone, 1,2-benzanthraquinone, 2-methylanthraquinone, 2-ethylanthraquinone, 1-dimethylaminoanthraquinone, 2,3-dimethylanthraquinone, 2-tert-butylanthraquinone, 1-chloroanthraquinone, 2-chloroanthraquinone, 1,5-dichloroanthraquinone, 1,2-dimethoxyanthraquinone, 1,2-diacetoxyanthraquinone, 5,12-naphthacenequinone, 6,13-pentacenequinone, xanthone, thioxanthone, 2,4-dimethylthioxanthone, 2,4-diethylthioxanthone, 2-chlorothioxanthone, 9(10H)-acridone, 9-methyl-9(10H)-acridone and benzosuberenone.

As the α-diketone compound, there can be exemplified camphorquinone, benzyl, diacetyl, acetylbenzoyl, 2,3-pentadion, 2,3-octadion, 4,4'-dimethoxybenzyl, 4,4'-oxybenzyl, 9,10-phenanthrenequinone, and acenaphthenequinone.

As the ketocoumarin compound, there can be exemplified 3-benzoylcoumarin, 3-(4-methoxybenzoyl)coumarin, 3-benzoyl-7-methoxycoumarin, 3-(4-methoxybenzoyl)-7-methoxy-3-coumarin, 3-acetyl-7-dimethylaminocoumarin, 3-benzoyl-7-dimethylaminocoumarin, 3,3'-coumarinoketone, and 3,3'-bis(7-diethylaminocoumarino)ketone.

As the self-cleaving radical-generating compound, there can be exemplified trichloroacetophenone.

As the electron-abstracting radical-generating compound, there can be exemplified xanthene compound, acridine compound and phenadine compound. As the xanthene compound, there can be exemplified Eosine compound, erythrosine compound and Rose Bengale. As the acridine compound, there can be exemplified acridine, 9-phenylacridine, benz[a]acridine and acridine orange. As the phenadine compound, there can be exemplified phenadine and benz[a]phenadine.

Among the above-mentioned various photo oxidation radical-generating compounds according to the present invention, it is desired to use a compound that absorbs visible light and, particularly, to use a compound having a maximum absorption wavelength in a range of 350 to 800 nm from the standpoint of obtaining a high degree of activity upon the irradiation with visible light that is generally used in the dental applications. It is, further, desired to use a hydrogen-abstracting photo radical-generator from the standpoint of a higher polymerization activity than those of other compounds when irradiated with light. In particular, it is desired to use a diarylketone compound, an α-diketone compound or a ketocoumarin compound.

The above-mentioned photo oxidation radical-generating compound (B) is used in an amount of 0.001 to 20 mols and, particularly, 0.005 to 10 mols per mole of the component (A).

### (C) Fused polycyclic aromatic compounds.

In the photopolymerization initiator of the present invention, the fused polycyclic aromatic compound (C) works as a so-called photosensitizer, has a fused aromatic ring, such as naphthalene ring, anthracene ring or phenanthrene ring, formed by the fusion of a plurality of aromatic rings, and has a molecular structure in which at least one aromatic carbon atom (hereinafter, called "adjacent carbon atom") adjacent to a ring-fusion carbon atom (this carbon atom shared by a plurality of rings) is bonded to a saturated carbon atom having at least one hydrogen atom. Here, the adjacent carbon atom constitutes the fused aromatic ring.

In the fused polycyclic aromatic compound, the adjacent carbon atoms bonded to the saturated carbon atom are for example, the carbon atoms at 1-, 4-, 5- and 8-positions when the fused aromatic ring is, a naphthalene ring, are the carbon atoms at 1-, 4-, 5-, 8-, 9- and 10-positions when the fused aromatic ring is an anthracene ring, and are the carbon atoms at 1-, 4-, 5-, 8-, 9- and 10-positions when the fused aromatic ring is a phenanthrene ring.

There is no particular limitation on the fused aromatic ring possessed by the fused polycyclic aromatic compound. Namely, the fused aromatic ring may be the one formed by the fusion of hydrocarbon rings only, such as naphthalene ring, anthracene ring, phenanthrene ring, or azulene ring, may be the one formed by the fusion of heterocyclic rings, such as naphthylidine ring, may be the one formed by the fusion of a hydrocarbon ring and a heterocyclic ring, such as quinoline ring. The fused aromatic ring may be the one formed by the fusion of two or more rings. From the standpoint of polymerization activity and easy availability, it is desired that the fused aromatic ring has at least one hydrocarbon ring (i.e., the one formed by the fusion of the hydrocarbon rings only or the one formed by the fusion of the hydrocarbon ring and the heterocyclic ring). More desirably, the fused aromatic ring is formed by the fusion of the hydrocarbon rings only and, particularly, is formed .by the fusion of the benzene rings only. On account of the same reasons, it is desired that the number of the aromatic rings that are fused is from 2 to 6 and, particularly, from 3 to 6. When there is used a compound having a monocycric aromatic ring (e.g. non-fused benzene ring or non-fused pyridine ring only) which is not a fused aromatic ring, it is not allowed to improve the polymerization activity, and the effect of the invention is not obtained.

Further, the saturated carbon atom having at least one hydrogen atom may exist in any form so far as it is bonded to the adjacent carbon atom of the fused aromatic ring. For example, the saturated carbon atom having at least one hydrogen atom may exist as part of a substituent such as an alkyl group bonded to the fused aromatic ring (e.g., methylanthracene or dichloromethylanthracene) or may exist as part of a non-aromatic ring which is further fused with the fused aromatic ring (e.g., acenaphthene or aceanthrene).

A representative example of the substituent bonded to the fused aromatic ring may be an unsubstituted or substituted alkyl group. Examples of the unsubstituted alkyl group include methyl group, ethyl group, propyl group, isopropyl group, butyl group, sec-butyl group, pentyl group, isopentyl group and hexyl group, having 1 to 20 carbon atoms. Further, concrete examples of the substituted alkyl group include alkoxyalkyl groups having 1 to 20 carbon atoms, such as methoxymethyl group, 1-methoxyethyl group, 1-methoxypropyl group, dimethoxymethyl group and diethoxymethyl group; arylalkyl groups having 7 to 20 carbon atoms, such as phenylmethyl group, p-tolylmethyl group, 1-phenylethyl group, 1-phenylpropyl group, diphenylmethyl group and bis(p-tolyl)methyl group; alkenylalkyl groups having 3 to 20 carbon atoms, such as diallylmethyl group and dimethallylmethyl group; hydroxyalkyl groups having 1 to 20 carbon atoms, such as hydroxymethyl group, 1-hydroxyethyl group and 1-hydroxypropyl group; halogenoalkyl groups having 1 to 20 carbon atoms, such as chloromethyl group, bromomethyl group, dichloromethyl group, dibromomethyl group, 1-chloroethyl group and 1-chloropropyl group; acyloxyalkyl groups having 2 to 20 carbon atoms, such as acetoxymethyl group, benzoyloxymethyl group, diacetyloxymethyl group, 1-acetyloxyethyl group and 1-acetyloxypropyl group; alkylthioalkyl groups having 1 to 20 carbon atoms, such as ethylthiomethyl group, butylthiomethyl group, 1-ethylthioethyl group and 1-butylthioethyl group; and mercaptoalkyl groups having 1 to 20 carbon atoms, such as mercaptomethyl group, 1-mercaptoethyl group and 1-mercaptopropyl group. Further, alkenyl groups having 3 to 20 carbon atoms, such as allyl group, methallyl group, 1-methylallyl group and 1-methylmethallyl group, can be exemplified as substituents in which the above-mentioned saturated carbon atom can exist.

When the saturated carbon atom exists as part of the non-aromatic ring which is further fused with the fused aromatic ring, the mode of fusion may be either the ortho fusion or the orthoperi fusion. As the non-aromatic ring, there can be exemplified saturated hydrocarbon ring, unsaturated hydrocarbon ring and heterocyclic ring having a hetero atom such as oxygen, nitrogen or sulfur.

As the saturated hydrocarbon ring, there can be exemplified cyclobutane ring, cyclopentane ring, cyclohexane ring, cycloheptane ring and cyclooctane ring. As the unsaturated hydrocarbon ring, further, there can be exemplified cyclopentene ring, cyclohexene ring, cycloheptene ring, 1,2-cycloheptadiene ring, cyclooctene ring, 1,2-cyclooctadiene ring and 1,3-cyclooctadiene ring. As the heterocyclic ring, there can be exemplified oxygen-containing rings such as oxetane ring, tetrahydrofurane ring and tetrahydropyran ring; nitrogen-containing rings such as azetidine ring, pyrrolidine ring and piperazine ring; and sulfur-containing rings such as trimethylene sulfide ring and tetramethylene sulfide ring. The names of these rings are those in a state where they have not been fused with the above-mentioned fused aromatic rings, and may often change as they are fused with the fused aromatic rings. Further, these non-aromatic rings may be so fused that the saturated carbon atom (having at least one hydrogen atom) in the ring is bonded to a carbon atom adjacent to the ring-fusion carbon atom in the fused aromatic ring. Further, the non-aromatic rings that have an unsaturated bond may be so fused as to share the unsaturated bond with the fused aromatic ring, or may be so fused as to exist as a part separate from the fused aromatic ring.

In the polycyclic fused aromatic compound used in the present invention, when the saturated carbon atom exists as an unsubstituted or substituted alkyl group, it is desired that the unsubstituted or substituted alkyl group has 1 to 10 carbon atoms. Further, when the saturated carbon atom exists as part of the non-aromatic ring fused with the fused aromatic ring, it is desired that the non-aromatic ring is a 5- to 7-membered ring (including atoms shared as atoms constituting the fused polycyclic aromatic ring) and, more preferably, a 5- to 7-membered non-aromatic hydrocarbon ring.

When used a compound in which no substituent has been bonded to the fused aromatic ring or a compound in which no non-aromatic ring has been fused with the fused aromatic ring, there is no improvement in the polymerization activity. Further, even by using a compound in which a substituent is bonded to the fused aromatic ring or a compound in which a non-aromatic ring is fused with the fused aromatic ring, there exists no saturated carbon atom when an unsaturated carbon atom such as vinyl group or phenyl group is bonded to the fused aromatic ring or when there exists only a group bonded with an atom other than carbon, such as methoxy group. In this case, therefore, there is no improvement in the polymerization activity, either. Similarly, despite there exists a saturated carbon atom, there is no improvement in the polymerization activity when the carbon atom has no hydrogen atom such as of t-butyl group, trichloromethyl group or 1,1-dichloroethyl group (when all hydrogen atoms are substituted).

The saturated carbon atoms having at least one hydrogen atom may be bonded in a plural number to the fused polycyclic aromatic ring. However, at least one of them must be bonded to a carbon atom adjacent to the ring-fusion carbon atom of the fused aromatic ring. When such a saturated carbon atom is bonded to an atom different from the adjacent carbon atom of the fused aromatic ring, it is not allowed to accomplish the polymerization rate to a sufficient degree.

The fused aromatic ring of the fused polycyclic aromatic compound used in the present invention may have a substituent such as hydroxyl group, halogen atom, mercapto group, t-butyl group or trichloromethyl group.

In the present invention, a particularly preferred fused polycyclic aromatic compound is represented by the following general formula (1),

R¹R²CₛₐₜH-A-(R³)ₙ (1)

wherein n is an integer of 0 to 6,
A is an aromatic hydrocarbon group having a valency of (n + 1) and with which 2 to 6 benzene rings are fused,
Cₛₐₜ is a saturated carbon atom bonded to a carbon atom adjacent to the ring-fusion carbon atom of the aromatic hydrocarbon group,
R¹ and R² are, independently from each other, hydrogen atoms, halogen atoms, hydroxyl groups, mercapto groups or monovalent organic residues having 1 to 10 carbon atoms, and
R³ is a halogen atom, a hydroxyl group, a mercapto group or a monovalent organic residues having 1 to 10 carbon atoms, and when n is 2 or more, R³s may be different from each other, and
wherein any two groups selected from R¹, R² and R³ may be bonded together, or two R³ groups may be bonded together to form a non-aromatic ring.

### [-Group A-]

In the above general formula (1), examples of the fused aromatic ring with which are fused 2 to 6 benzene rings possessed by the aromatic hydrocarbon group A of a valency of (n + 1) include a naphthalene ring fused with 2 benzene rings; an anthracene ring or a phenanthrene ring fused with 3 benzene rings; a naphthacene ring, a 1,2-benzanthracene ring, a chrysene ring or a pyrene ring fused with 4 benzene rings; a benzo(a)pyrene ring, a benzo(e)pyrene ring, a benzo(g)pyrene ring, a benzo(h)pyrene ring, a benzo(i)pyrene ring, a perylene ring, a pentacene ring, a pentaphene ring and a picene ring fused with 5 benzene rings; and hexaphene ring and hexacene ring fused with 6 benzene rings.

### [Groups R¹ and R²]

The groups R¹ and R² are hydrogen atoms, halogen atoms, hydroxyl groups, mercapto groups or monovalent organic residues having 1 to 10 carbon atoms. As the halogen atom, there can be exemplified a fluorine atom, a chlorine atom, a bromine atom or an iodine atom. As the monovalent organic residue having 1 to 10 carbon atoms, there can be exemplified a substituted or unsubstituted alkyl group, a substituted or unsubstituted alkoxy group, a substituted or unsubstituted alkylthio group, a substituted or unsubstituted aryl group, a substituted or unsubstituted alkenyl group, acyloxy group or a dialkylamino group.

In the above monovalent organic residue, examples of the unsubstituted alkyl group include alkyl groups having 1 to 10 carbon atoms, such as methyl group, ethyl group, propyl group, isopropyl group, butyl group, sec-butyl group, t-butyl group, pentyl group, isopentyl group and hexyl group. Though there is no particular limitation on the substituent of the substituted alkyl group in the monovalent organic residue, its concrete examples include alkoxy groups such as methoxy group and ethoxy group; aryl groups such as phenyl group and tolyl group; 1-alkenyl groups such as vinyl group and 1-propenyl group; hydroxyl group; halogen atoms such as fluorine atom, chlorine atom and bromine atom; acyloxy groups such as acetyloxy group and benzoyloxy group; alkylthio groups such as ethylthio group and butylthio group; and mercapto group. Therefore, concrete examples of the substituted alkyl group having such substituents include alkoxyalkyl groups having 1 to 10 carbon atoms, such as methoxymethyl group, 1-methoxyethyl group, 1-methoxypropyl group, dimethoxymethyl group, and diethoxymethyl group; arylalkyl groups having 7 to 10 carbon atoms, such as phenylmethyl group, p-tolylmethyl group, 1-phenylethyl group and 1-phenylpropyl group; alkenyl groups having 3 to 10 carbon atoms, such as allyl group, methallyl group, 1-methylallyl group and 1-methylmethallyl group; alkenylalkyl groups having 3 to 10 carbon atoms, such as allyl group (vinylmethyl group), diallylmethyl group and dimethallylmethyl group; hydroxyalkyl groups having 1 to 10 carbon atoms, such as hydroxymethyl group, 1-hydroxyethyl group and 1-hydroxypropyl group; halogenoalkyl groups having 1 to 10 carbon atoms, such as chloromethyl group, bromomethyl group, dichloromethyl group, trichloromethyl group, dibromomethyl group, 1-chloroethyl group and 1-chloropropyl group; acyloxyalkyl groups having 2 to 10 carbon atoms, such as acetoxymethyl group, benzoyloxymethyl group, diacetyloxymethyl group, 1-acetyloxyethyl group and 1-acetyloxypropyl group; alkyl-thioalkyl groups having 2 to 10 carbon atoms, such as ethylthiomethyl group, butylthiomethyl group, 1-ethylthioethyl group and 1-butylthioethyl group; and mercaptoalkyl groups such as mercaptomethyl group, 1-mercaptoethyl group and 1-mercaptopropyl group.

As the substituted or unsubstituted alkoxy group, or substituted or unsubstituted alkylthio group in the monovalent organic residue, there can be exemplified alkoxy group or alkylthio group derived from the substituted or unsubstituted alkyl group (e.g., methoxy group and methylthio group are derived from the methyl group) and, preferably, alkoxy group and alkylthio group having 1 to 10 carbon atoms.

As the substituted or unsubstituted aryl group in the monovalent organic residue, there can be exemplified aryl groups having 6 to 10 carbon atoms, such as phenyl group, naphthyl group, tolyl group and xylyl group. As the substituted or unsubstituted alkenyl group, there can be exemplified alkyl groups having 2 to 10 carbon atoms, such as vinyl group and 1-propenyl group. As the acyloxy group, there can be exemplified acyloxy groups having 1 to 10 carbon atoms, such as acetoxy group and benzoyloxy group. As the dialkylamino group, there can be exemplified dialkylamino groups having 2 to 10 carbon atoms, such as dimethylamino group and diethylamino group.

Among the groups (R¹R²CₛₐₜH-) having the above R¹ and R² in the general formula (1), it is desired to use the one having not more than 20 carbon atoms and, particularly, not more than 10 carbon atoms from the standpoint of polymerization activity. Concrete examples of the above group include alkyl groups having 1 to 10 carbon atoms, such as methyl group, ethyl group, propyl group, isopropyl group, butyl group, sec-butyl group, pentyl group, isopentyl group and hexyl group; alkoxyalkyl groups having 1 to 10 carbon atoms, such as methoxymethyl group, 1-methoxyethyl group, 1-methoxypropyl group, dimethoxymethyl group and diethoxymethyl group; arylalkyl groups having 7 to 15 carbon atoms, such as phenylmethyl group, p-tolylmethyl group, 1-phenylethyl group, 1-phenylpropyl group, diphenylmethyl group and bis(p-tolyl)methyl group; alkenyl group having 3 to 10 carbon atoms, such as allyl group, methallyl group, 1-methylallyl group, and 1-methylmethallyl group, diallylmethyl group and dimethallylmethyl group; hydroxyalkyl group having 1 to 10 carbon atoms, such as hydroxymethyl group, 1-hydroxyethyl group and 1-hydroxypropyl group; halogenoalkyl groups having 1 to 10 carbon atoms, such as chloromethyl group, bromomethyl group, dichloromethyl group, dibromomethyl group, 1-chloroethyl group and 1-chloropropyl group; acyloxyalkyl groups having 2 to 10 carbon atoms, such as acetoxymethyl group, benzoyloxymethyl group, diacetyloxymethyl group, 1-acetyloxyethyl group and 1-acetyloxypropyl group; alkylthioalkyl groups having 1 to 10 carbon atoms, such as ethylthiomethyl group, butylthiomethyl group, 1-ethylthioethyl group and 1-butylthioethyl group; and mercaptoalkyl groups having 1 to 10 carbon atoms, such as mercaptomethyl group, 1-mercaptoethyl group and 1-mercaptopropyl group.

The above group (R¹R²CₛₐₜH-) must have been bonded to the carbon atom adjacent to the ring-fusion carbon atom of the fused aromatic ring possessed by the aromatic hydrocarbon group A.

### [-R³-]

In the general formula (1), R⁷ is a halogen atom, a hydroxyl group, a mercapto group or a monovalent organic residue having 1 to 10 carbon atoms, and its concrete examples are the same as those described concerning R¹ and R². When n is 2 to 6, a plurality of R³ groups may be the same or different.

Further, the group R³ may be bonded to any position of the fused polycyclic aromatic hydrocarbon ring denoted by A.

Any two groups selected from R¹, R² and R³ may be bonded together, or a plurality of R³ groups may be bonded together to form a non-aromatic ring. The non-aromatic rings may exist in a plural number. In this case, the plurality of non-aromatic rings may be different from each other.

Concrete examples of the non-aromatic ring formed by the groups R¹ and R² bonded together include cyclopropane ring, cyclobutane ring, cyclopentane ring, cyclohexane ring, tetrahydropyrane ring and tetrahydrofuran ring.

Further, the non-aromatic ring formed by the group R¹ or R² and the group R³, or the non-aromatic ring formed by a plurality of R³ groups bonded together, forms a non-aromatic ring fused with the fused aromatic ring possessed by the aromatic hydrocarbon group A. There is no particular limitation on the non-aromatic ring, and either the hydrocarbon ring or the heterocyclic ring may be used. Concrete examples thereof are the same as those of the case where the saturated carbon atom (Cₛₐₜ) having at least one hydrogen atom exists as part of the non-aromatic ring fused with the fused polycyclic aromatic ring.

Concrete examples of the fused polycyclic aromatic compound represented by the above general formula (1) that can be preferably used in the present invention are as described below.

### [Compounds in which the groups R¹ to R³ are existing independently]

Naphthalene derivatives such as 1-methylnaphthalene, 1-ethylnaphthalene and 1,4-dimethylnaphthalene;
phenanthrene derivatives such as 4,5-dimethylphenanthrene and 1,8-dimethylphenanthrene;
anthracene derivatives such as 1-methylanthracene, 9-methylanthracene, 9-ethylanthracene, 9,10-dimethylanthracene, 9,10-diethylanthracene, 9-methoxymethylanthracene, 9-(1-methoxyethyl)anthracene, 9-hexyloxymethylanthracene, 9,10-dimethoxymethylanthracene, 9-dimethoxymethylanthracene, 9-phenylmethylanthracene, 9-(1-naphthyl)methylanthracene, 9-hydroxymethylanthracene, 9-(1-hydroxyethyl)anthracene, 9,10-bis(hydroxymethyl)anthracene, 9-acetoxymethylanthracene, 9-(1-acetoxyethyl) anthracene, 9,10-bis(acetoxymethyl)anthracene, 9-benzoyloxymethylanthracene, 9,10-dibenzoyloxymethylanthracene, 9-ethylthiomethylanthracene, 9-(1-ethylthioethyl)anthracene, 9,10-bis(ethylthiomethyl)anthracene, 9-mercaptomethylanthracene, 9-(1-mercaptoethyl)anthracene, 9,10-bis(mercaptomethyl)anthracene, 9-ethylthiomethyl-10-methylanthracene, 9-methyl-10-phenylanthracene, 9-methyl-10-vinylanthracene, 9-allylanthracene, 9,10-diallylanthracene, 9-chloromethylanthracene, 9-bromonethylanthracene, 9-iodomethylanthracene, 9-(1-chloroethyl)anthracene, 9-(1-bromoethyl)anthracene, 9-(1-iodoethyl)anthracene, 9,10-bis(chloromethyl)anthracene, 9,10-bis(bromomethyl)anthracene, 9,10-bis(iodomethyl)anthracene, 9-chloro-10-methylanthracene, 9-chloro-10-ethylanthracene, 9-bromo-10-methylanthracene, 9-bromo-10-ethylanthracene, 9-iodo-10-methylanthracene, 9-iodo-10-ethylanthracene and 9-methyl-10-(dimethylamino)anthracene, and derivatives of fused polycyclic aromatic hydrocarbons in which are fused 4 to 6 benzene rings, such as 7,12-dimethylbenz(a)anthracene, 7,12-dimethoxymethylbenz(a)anthracene, 5,12-dimethylnaphthacene, 7-methylbenzo(a)perylene, 3,4,9,10-tetramethylperylene, 3,4,9,10-tetrakis(hydroxymethyl)perylene, 6,13-dimethylpentacene, 8,13-dimethylpentaphene, 5,16-dimethylhexacene and 9,14-dimethylhexaphene.

### [Compounds in which R¹ and R² are bonded together to form a ring]

9-Cyclohexylanthracene, 9,10-dicyclohexylanthracene, etc.

### [Compounds in which R¹ or R² and R³ are bonded together to form a ring]

Acenaphthene, phenalene, acephenanthrene, aceanthrene, acenaphthenone, 1,2,3,4-tetrahydrophenanthrene, benzo[e]phthalane, benzo[f]coumaron, benzo[f]isocoumaron, N-methylbenzo[e]indolin, N-methylbenzo[e]isoindolin, cholanthrene, violanthrene and isoviolanthrene.

The above-mentioned fused polycyclic aromatic compounds (C) are used in one kind or being mixed in two or more kinds together, and the amount of use thereof is, usually, 0.0005 to 20 mols and, particularly, 0.001 to 20 mols per mol of the component (A)

The photopolymerization initiator of the present invention containing the above-mentioned components (A) to (C) are capable of efficiently polymerizing both the cationically polymerizable monomer and the radically polymerizable monomer. The polymerization initiator capable of efficiently photopolymerizing both the cationically polymerizable monomer and the radically polymerizable monomer has not heretofore been known but was discovered, for the first time, by the present inventors.

### (Photopolymerizable composition)

The photopolymerizable composition of the present invention is a combination of the above-mentioned photopolymerization initiator and the polymerizable monomer. As the polymerizable monomer, there can be used either a known cationically polymerizable monomer or a known radically polymerizable monomer owing to the properties of the photopolymerization initiator.

Concrete examples of the representative cationically polymerizable monomer include a vinyl ether compound, an epoxy compound, an oxetane compound, an aziridine compound, an azetidine compound, an episulfide compound, a cyclic acetal, a bicycloortho ester, a spiroortho ester, a spiroortho carbonate and a tetrahydrofurane. When the dental applications are taken into consideration, in particular, it is desired to use the oxetane compound and the epoxy compound, since they are easily available, show low volumetric shrinkage, and undergo a quick polymerization reaction.

Concrete examples of the oxetane compound include those compounds having one oxetane ring, such as trimethylene oxide, 3-methyl-3-oxetanyl methanol, 3-ethyl-3-oxetanyl methanol, 3-ethyl-3-phenoxymethyl oxetane, 3,3-diethyl oxetane, and 3-ethyl-3-(2-ethylhexyloxy)oxetane; compounds having two or more oxetane rings, such as 1,4-bis(3-ethyl-3-oxetanylmethyloxy)benzene, 4,4'-bis(3-ethyl-3-oxetanylmethyloxy)biphenyl, 4,4'-bis(3-ethyl-3-oxetanylmethyloxymethyl)biphenyl, ethylene glycol bis(3-ethyl-3-oxetanylmethyl)ether, diethylene glycol bis(3-ethyl-3-oxetanylmethyl)ether, bis(3-ethyl-3-oxetanylmethyl)diphenoate, trimethylolpropanetris(3-ethyl-3-oxetanylmethyl)ether, pentaerythritoltetrakis(3-ethyl-3-oxetanylmethyl)ether, as well as compounds represented by the following formulas,

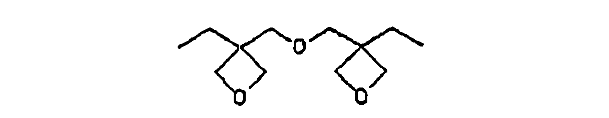

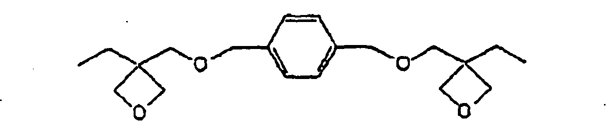

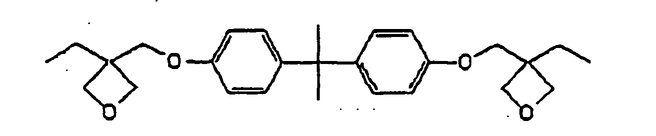

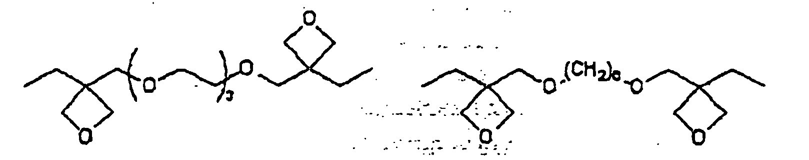

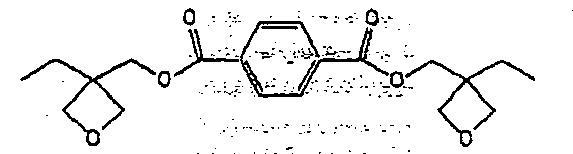

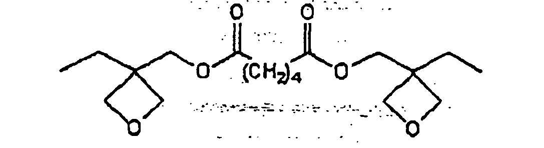

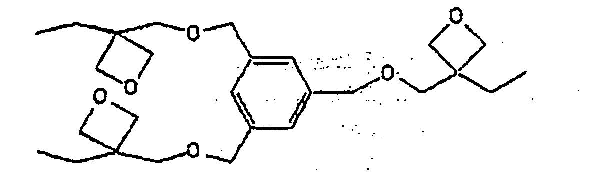

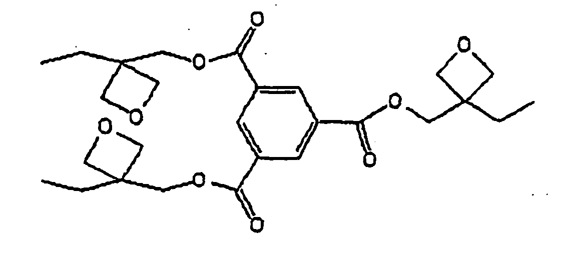

In the present invention, there are used those compounds having two or more oxetane rings in one molecule of the monomer from the standpoint of properties of the obtained cured material.

Examples of the epoxy compound that can be preferably used as the cationically polymerizable monomer include diglycerolpolydiglycidyl ether, pentaerythritolpolyglycidyl ether, 1,4-bis(2,3-epoxypropoxyperfluoroisopropyl)cyclohexane, sorbitolpolyglycidyl ether, trimethylolpropanepolyglycidyl ether, resorcindiglycidyl ether, 1,6-hexanedioldiglycidyl ether, polyethylene glycol diglycidyl ether, phenylglycidyl ether, p-tert-butylphenylglycidyl ether, diglycidyl adipate ester, o-diglycidyl phthalate ester, dibromophenylglycidyl ether, 1,2,7,8-diepoxyoctane, 4,4'-bis(2,3-epoxypropoxyperfluoroisopropyl)diphenyl ether, 2,2-bis[4-glycidyloxyphenyl]propane, 3,4-epoxycyclohexylmethyl-3',4'-epoxycyclohexane carboxylate, 3,4-epoxycyclohexyloxysilane, and ethylene glycol-bis(3,4-epoxycyclohexane carboxylate).

These cationically polymerizable monomers can be used alone or in a combination of two or more kinds.

Examples of the radically polymerizable monomer that can be preferably used include (meth)acrylate monomers having one (meth)acryloxy group, such as methyl(meth)acrylate, ethyl(meth)acrylate, isopropyl(meth)acrylate, butyl(meth)acrylate, 2-ethylhexyl(meth)acrylate, benzyl (meth) acrylate, polyethylene glycol mono(meth)acrylate, 2-hydroxyethyl(meth)acrylate, 3-hydroxypropyl(meth)acrylate, glycelyl mono(meth)acrylate, tetrahydrofurfuryl(meth)acrylate, 2-(meth)acryloxyethyl propionate, 2-methacryloxyethylaceto acetate and allyl(meth)acrylate; (meth)acrylate monomers having a plurality of (meth)acryloxy groups, such as ethylene glycol di(meth)acrylate, diethylene glycol di(meth)acrylate, triethylene glycol di(meth)acrylate, butylene glycol di(meth)acrylate, nonaethylene glycol di(meth)acrylate, polyethylene glycol di(meth)acrylate, propylene glycol di(meth)acrylate, dipropylele glycol di(meth)acrylate, neopentyl glycol di(meth)acrylate, 1,3-butanediol di(meth)acrylate, 1,3-hexanediol di(meth)acrylate, 1,4-butanediol di(meth)acrylate, 1,6-hexanediol di(meth)acrylate, 1,9-nonanediol di (meth) acrylate, trimethylolpropane tri(meth)acrylate, pentaerythritol tri(meth)acrylate, trimethylolmethane tri(meth)acrylate, pentaerythritol tetra(meth)acrylate, urethane (meth)acrylate, 2,2-bis((meth)acryloxyphenyl)propane, 2,2-bis[4-(2-hydroxy 3-(meth)acryloxy)propoxyphenyl]propane, 2,2-bis(4-(meth) acryloxyethoxyphenyl) propane, 2,2-bis(4-(meth)acryloxydiethoxyphenyl)propane and 2,2-bis(4-(meth)acryloxypropoxyphenyl)propane; (meth)acrylamide monomers such as diacetoneacrylamide and N-methylol (meth)acrylamide; fumaric acid ester monomers such as dimethyl fumarate, diethyl fumarate and diphenyl fumarate; styrene monomers such as styrene, divinylbenzene and α-methylstyrene; allyl monomers such as diallyl phthalate, diallyl terephthalate, diallyl carbonate and allyl diglycol carbonate; and vinyl acetate, 4-vinylpyridine, N-vinylpyrrolidone and ethylvinyl ether.

When the polymerizable composition of the present invention is used for dental applications, there can, as required, be used radically polymerizable monomers having an acidic group, i.e., radically polymerizable monomers having one carboxyl group in the molecules as well as acid anhydrides thereof and acid halides thereof, such as (meth)acrylic acid, N-(meth)acryloylglycine, N-(meth)acryloylaspartic acid, N-(meth)acryloyl-5-aminosalicilic acid, 2-(meth)acryloyloxyethyl hydrogensuccinate, 2-(meth)acryloyloxyethyl hydrogenphthalate, 2-(meth)acryloyloxyethyl hydrogen maleate, 6-(meth)acryloyloxyethylnaphthalene-1,2,6-tricarboxylic acid, O-(meth)acryloyltyrosine, N-(meth)acryloyltyrosine, N-(meth)acryloylphenylalanine, N-(meth)acryloyl-p-aminobenzoic acid, N-(meth)acryloyl-o-aminobenzoic acid, p-vinylbenzoic acid, 2-(meth) acryloyloxybenzoic acid, 3-(meth)acryloyloxybenzoic acid, 4-(meth) acryloyloxybenzoic acid, N- (meth) acryloyl-5-aminosalicilic acid, N-(meth)acryloyl-4-aminosalicilic acid; radically polymerizable monomers having a plurality of carboxyl groups or acid anhydride groups thereof in the molecules, such as 11-(meth)acryloyloxyundecane-1,1-dicarboxylic acid, 10-(meth)acryloyloxydecane-1,1-dicarboxylic acid, 12-(meth) acryloyloxydodecane-1,1-dicarboxylic acid, 6-(meth)acryloyloxyhexane-1,1-dicarboxylic acid, 2-(meth)acryloyloxyethyl-3'-methacryloyloxy-2'-(3,4-dicarboxybenzoyloxy)propyl succinate, 1,4-bis(2-(meth)acryloyloxyethyl)pyromellitate, N,O-di(meth)acryloyltyrosine, 4-(2-(meth)acryloyloxyethyl)trimellitic anhydride, 4-(2-(meth)acryloyloxyethyl)trimellitate, 4-(meth)acryloyloxyethyl trimellitate, 4-(meth)acryloyloxybutyl trimellitate, 4-(meth)acryloyloxyhexyl trimellitate, 4-(meth)acryloyloxydecyl trimellitate, 4-acryloyloxybutyl trimellitate, 6-(meth)acryloyloxyethylnaphthalene-1,2,6-tricarboxylic anhydride, 6-(meth)acryloyloxyethylnaphthalene-2,3,6-tricarboxylic anhydride, 4-(meth)acryloyloxyethylcarbonylpropionoyl-1,8-naphthalic anhydride, and 4-(meth)acryloyloxyethylnaphthalene-1,8-tricarboxylic anhydride; radically polymerizable monomers having a phosphinicooxy group or a phosphonooxy group in the molecules, such as 2-(meth)acryloyloxyethyl dihydrogenphosphate, bis(2-(meth)acryloyloxyethyl) hydrogenphosphate, 2-(meth)acryloyloxyethyl phenyl hydrogenphosphate,10-(meth)acryloyloxydecyl hydrogenphosphate, 6-(meth)acryloyloxyhexyl dihydrogenphosphate and 2-(meth)acryloyloxyethyl 2-bromoethyl hydrogenphosphate; radically polymerizable monomers having a phosphono group in the molecules, such as vinylphosphonic acid and p-vinylbenzenephosphonic acid; and radically polymerizable monomers having a sulfo group in the molecules, such as 2-(meth)acrylamide-2-methylpropanesulfonic acid, p-vinylbenzenesulfonic acid, and vinylsulfonic acid.

As the radically polymerizable monomer in the present invention, it is desired to use a (meth)acrylate monomer having one or a plurality of (meth)acryloxy groups from the standpoint of polymerization activity, or to use a (meth)acrylate monomer having a plurality of (meth)acryloxy groups from the standpoint of obtaining a cured material featuring a high strength.

The above-mentioned radically polymerizable monomers can be used in one kind or in a combination of two or more kinds.

In the present invention, it is desired to use the cationically polymerizable monomer in combination with the radically polymerizable monomer. When the cationically polymerizable monomer and the radically polymerizable monomer are used in combination, it is desired to also use a monomer having both a cationically polymerizing functional group and a radically polymerizing functional group, such as glycidyl (meth)acrylate.

When the cationically polymerizable monomer and the radically polymerizable monomer are to be used in combination, the ratio of the two may be suitably determined without any particular limitation. The volumetric shrinkage at the time of curing by polymerization, however, decreases with an increase in the cationically polymerizable monomer. It is therefore desired to use the radically polymerizable monomer in amounts of not larger than 200 parts by mass per 100 parts by mass of the cationically polymerizable monomer.

In the photopolymerizable composition of the present invention, the above-mentioned photopolymerization initiator is used in such an amount that the amount of the photo acid-generating compound (A) is 0.001 to 10 parts by mass and, particularly, 0.05 to 5 parts by mass per 100 parts by mass of the polymerizable monomer as described already.

In addition to being blended with the photopolymerization initiator and the polymerizable monomer, the polymerizable composition of the present invention can be further blended with a variety of known blending agents, such as known cationic polymerization initiator, radical polymerization initiator, filler, polymerization inhibitor, antioxidant, ultraviolet ray absorbent, dye, antistatic agent, pigment, perfume, organic solvent and viscosity-imparting agent.

As the cationic polymerization initiator of the present invention other than the photopolymerization initiator, there can be exemplified boron trifluoride ether complex, titanium tetrachloride, aluminum trichloride, p-toluenesulfonic acid, trifluoromethanesulfonic acid, hydrochloric acid, sulfuric acid, perchloric acid, iodine, iodine bromide and triphenylmethylhexafluoro antimonato.

As the radically polymerizable initiator, further, there can be exemplified peroxides such as benzoyl peroxide, p-chlorobenzoyl peroxide, tert-butylperoxy-2-ethyl hexanoate, tert-butylperoxy dicarbonate, diisopropylperoxy dicarbonate, dilauroyl peroxide, t-butyl hydroperoxide, cumene hydroperoxide, di t-butyl peroxide, dicumyl peroxide, and diacetyl peroxide; azo-compounds such as azobisisobutylonitrile, etc.; boron compounds such as tributylborane, partial oxide of tributylborane, sodium tetraphenylborate, sodium tetrakis(p-fluorophenyl)borate and potassium tetrakis (p-chlorophenyl)borate; sulfinic acids such as benzenesulfinic acid, p-toluenesulfinic acid, 2,3-dimethylbenzenesulfinic acid, 3,5-dimethylbenzenesulfinic acid and α-naphthalenesulfinic acid, or sulfinic acids (salts) such as alkali metal salts thereof; and barbituric acids such as 5-butyl(thio)barbituric acid, 1,3,5-trimethyl(thio)barbituric acid, 1-benzyl-5-phenyl(thio)barbituric acid, 1-cyclohexyl-5-methyl(thio)barbituric acid and 1-cyclohexyl-5-butyl(thio)barbituric acid. When these radical polymerization initiators are to be used, it is desired to also use known polymerization initiators such as various amine compounds and metal compounds in combination.

The polymerization initiators of the present invention other than the photopolymerization initiators can be used in one kind or in a combination of a plurality of kinds in combination, as required. These other polymerization initiators may be added in amounts lying in a known range and are, generally, added in amounts of not larger than 10 parts by mass and, desirably, not larger than 5 parts by mass per 100 parts by mass of the polymerizable monomers as a whole.

By further using arylamino compounds such as an alkyl (methyl, ethyl, isoamyl, etc.) N,N-dimethylaminobenzoate and an N,N-dimethylaminoacetophenone in combination, the polymerization activity of the photopolymerizable composition of the present invention can be further improved.

The photopolymerizable composition of the present invention by itself is useful as a surface coating material or an adhesive. By adding a filler thereto, its volumetric shrinkage due to the polymerization can be further decreased. Further, use of the filler improves the operability of the polymerizable composition of before being cured or improves the mechanical properties of after being cured enhancing the utility of the photopolymerizable composition as a dental-material and, particularly, as a filling restorative for dental use (dental composite resin).

As the filler, there can be used, without any particular limitation, a known filler that has been generally used for the dental composite resins. The fillers can generally be roughly divided into organic fillers and inorganic fillers.

Concrete examples of a representative organic filler that can be preferably used include polymethyl methacrylate, polyethyl methacrylate, methyl methacrylate/ethyl methacrylate copolymer, crosslinked polymethyl methacrylate, crosslinked polyethyl methacrylate, ethylene/vinyl acetate copolymer, styrene/butadiene copolymer, acrylonitrile/styrene copolymer, and acrylonitrile/styrene/butadiene copolymer, which can be used in one kind or as a mixture of two or more kinds.

Concrete examples of a representative inorganic filler include quartz, silica, alumina, silica titania, silica zirconia, lanthanum glass, barium glass, strontium glass and various cation-eluting fillers. As the cation-eluting filler, there can be exemplified hydroxides such as calcium hydroxide and strontium hydroxide, and oxides such as zinc oxide, silicate glass and fluoroaluminosiliate glass. The inorganic fillers, too, may be used in one kind or being mixed together in two or more kinds. Use of the inorganic filler containing a heavy metal such as zirconia contributes to imparting X-ray contrast property.

It is further allowable to use a granular organic/inorganic composite filler obtained by adding a polymerizable monomer to the inorganic fillers to obtain a paste thereof and, then, polymerizing the paste, and pulverizing a cured material.

It is further allowable to use the above organic filler, inorganic filler and organic/inorganic composite filler in a suitable combination.

There is no particular limitation on the particle size and shape of the filler. The filler having an average particle size of 0.01 µm to 100 µm, that is usually used as a dental material, can be suitably used depending on the object. There is no particular limitation, either, on the refractive index of the filler; i.e., the filler having a refractive index of 1.4 to 1.7 as possessed by dental fillers, in general, can be used without any limitation.

The ratio of blending the filler may be suitably determined depending upon the object of use by taking into consideration the viscosity (operability) of when it is mixed into the polymerizable monomer and the mechanical properties of the cured material. In general, however, the filler is blended in a range of 50 to 1500 parts by mass and, preferably, 70 to 1000 parts by mass per 100 parts by mass of the polymerizable monomer.

There is no particular limitation on the method of producing the photopolymerizable composition of the present invention, and any known method may be suitably employed for producing a cured composition by photopolymerization. Concretely speaking, the components constituting the polymerization initiator composition of the invention as well as other components that are blended as required, are weighed by predetermined amounts and are mixed together. Here, it is desired that the components are weighed and mixed while shielding light so that the curable composition will not be cured at the time of being mixed together.

There is no particular limitation on the form of packaging the photopolymerizable composition of the present invention; i.e., the packaging may be suitably determined by taking the object and preservation stability into consideration. In general, all components constituting the photopolymerizable composition of the present invention may be contained in a package (light-shielding package). When the cationically polymerizable monomer and the radically polymerizable monomer having an acidic group or any other cationically polymerizable initiator are to be blended, however, they are likely to be cured while being preserved. In such a case, therefore, it is desired that they are divided into two or more packages and are mixed together just before the use.

As means for curing the photopolymerizable composition of the present invention, there can be used such light sources as carbon arc, xenon lamp, metal halide lamp, tungsten lamp, fluorescent lamp, sunlight, helium-cadmium laser or argon laser without any limitation. The time of irradiation varies depending upon the wavelength of the source of light, intensity, shape and material of the cured material and may, hence, be determined in advance by preparatory experiment. In general, however, it is desired to adjust the rate of blending various components such that the time of irradiation lies in a range of about 5 to about 60 seconds.

### Examples

The invention will now be concretely described by way of Examples to which only, however, the invention is in no way limited. Described below are the compounds and their abbreviations used in the specification and in Example.

### (1) Cationically polymerizable monomers.

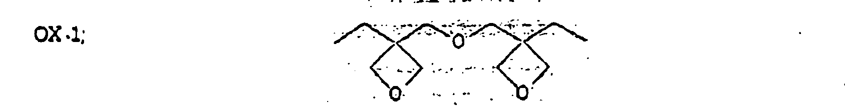

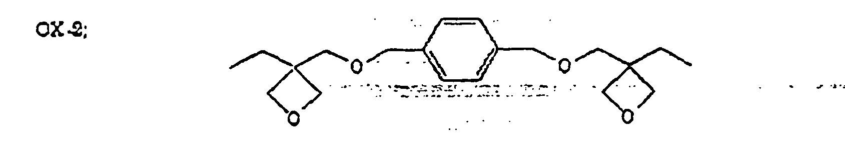

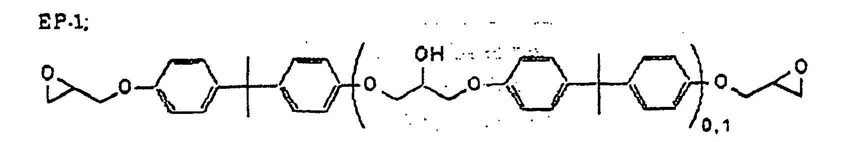

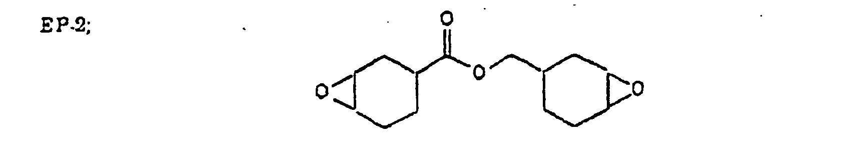

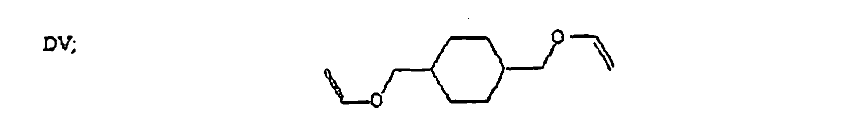

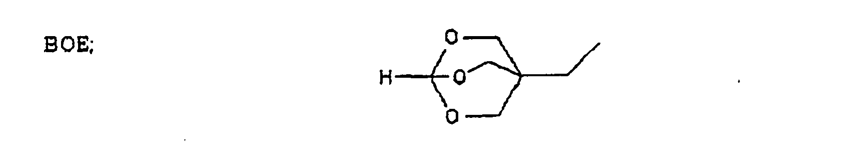

### (2) Radically polymerizable monomers.

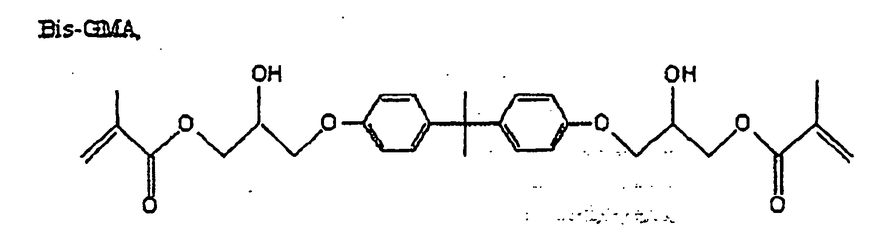

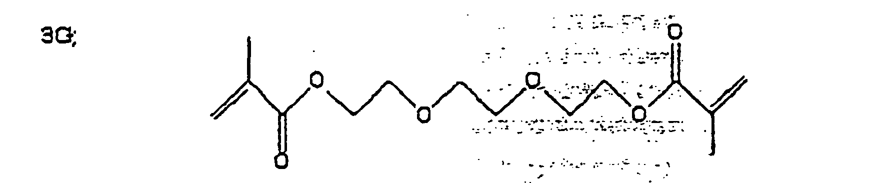

### (3) Photo acid-generating compounds (A).

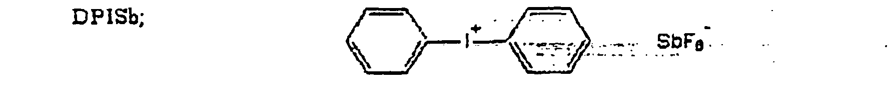

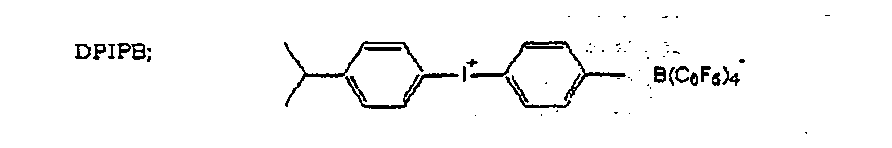

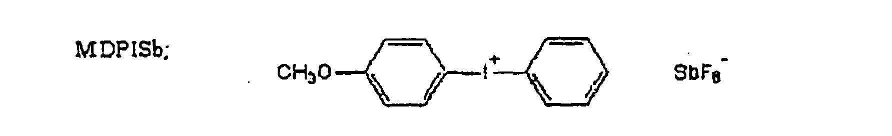

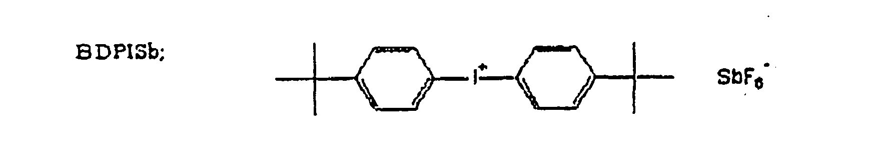

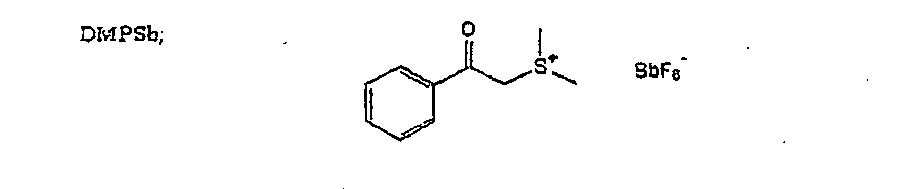

### (4) Diaryl ketone photo radical-generating compounds (B): numerals in [ ] represent maximum absorption wavelengths.

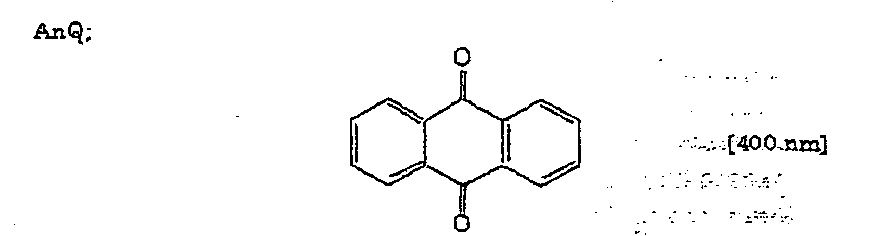

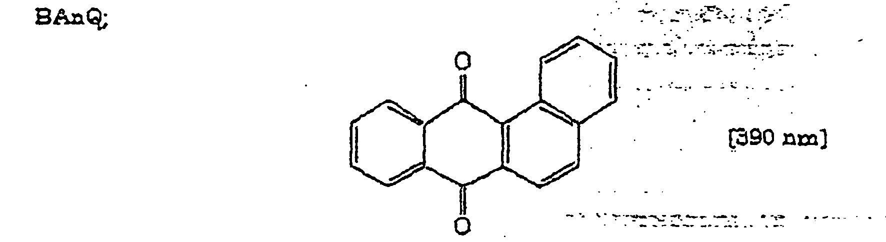

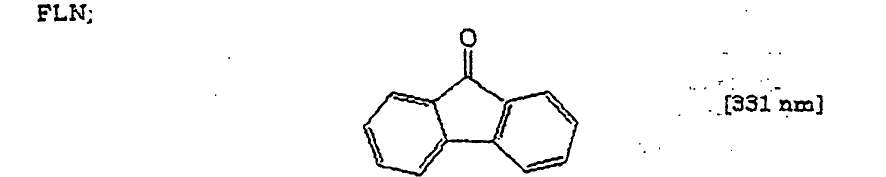

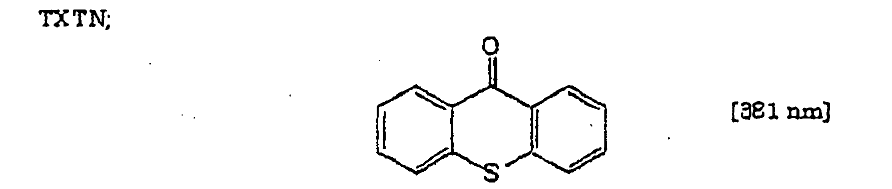

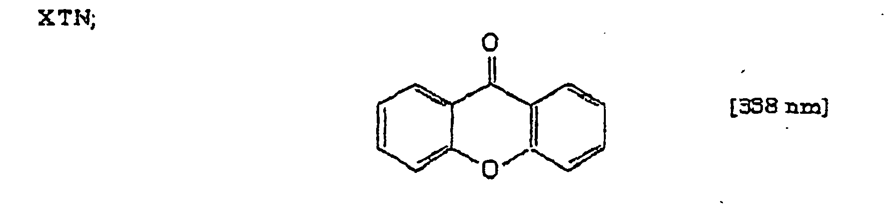

### (5) α-Diketone photo radical-generating compounds (B): numerals in [ ] represent maximum absorption wavelengths.

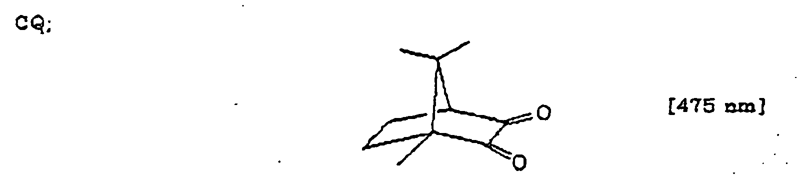

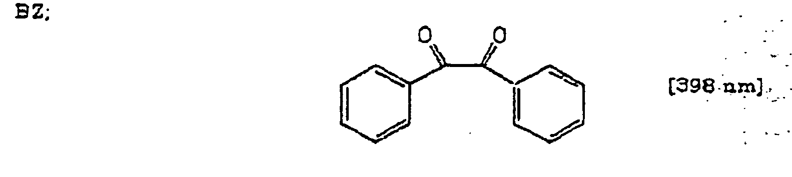

### (6) Ketocoumarin photo radical-generating compound (B) : numeral in [ ] represents a maximum absorption wavelength.

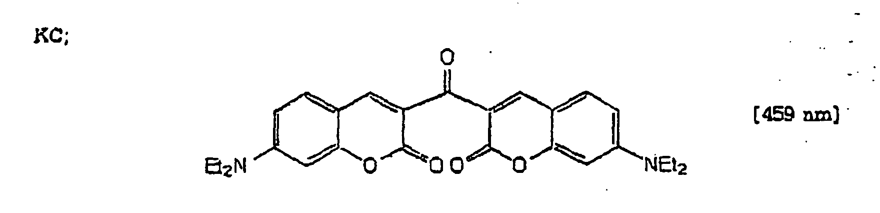

### (7) Fused polycyclic aromatic compounds (C) of the invention.

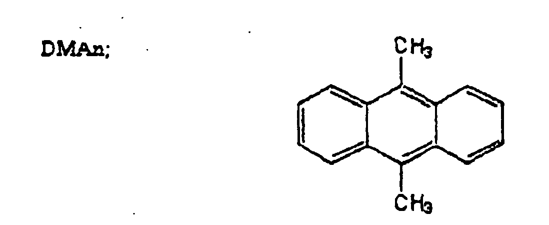

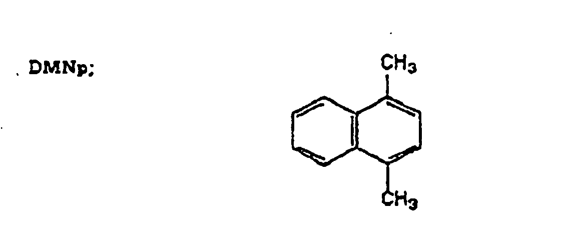

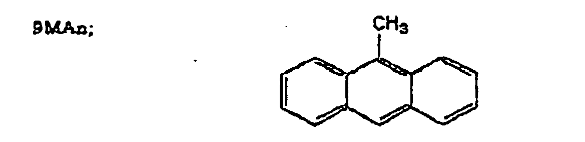

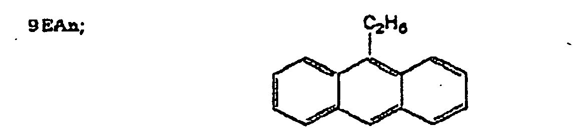

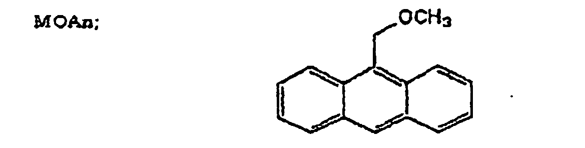

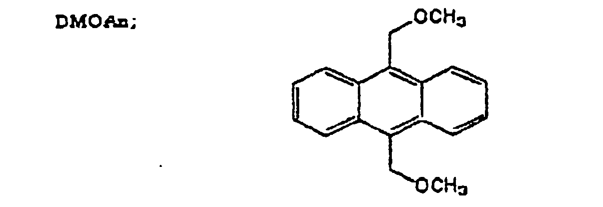

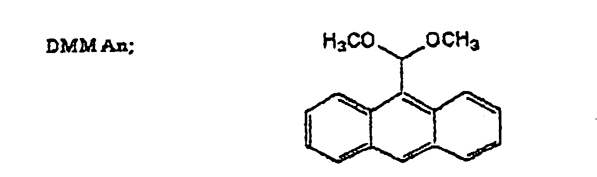

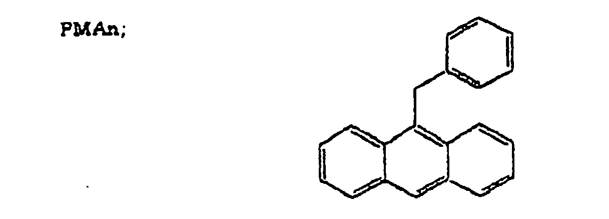

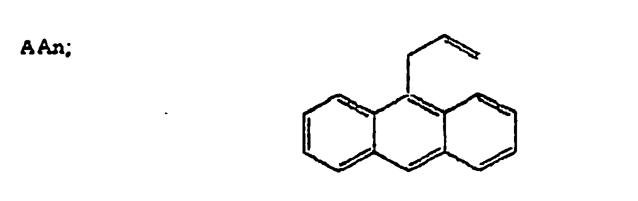

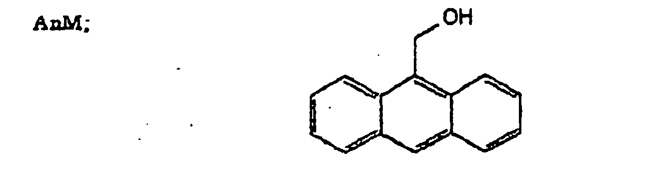

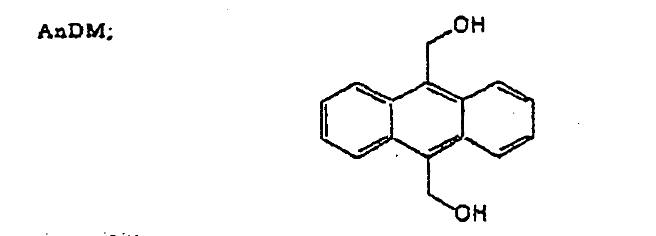

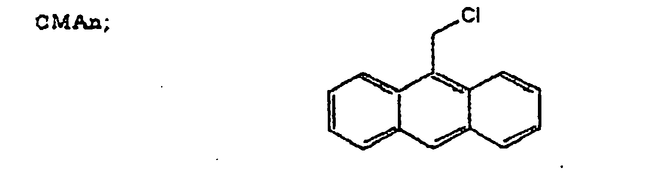

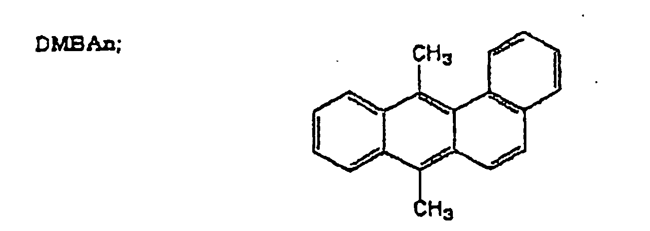

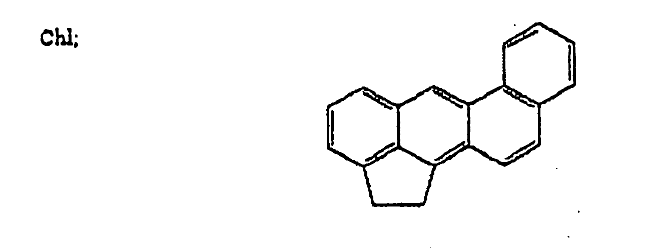

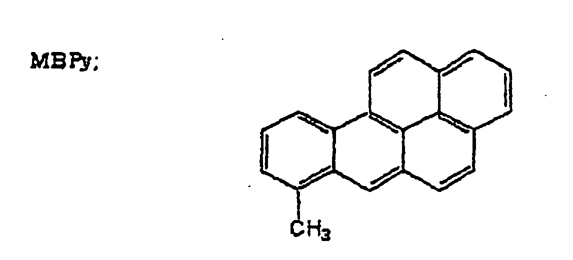

### (8) Other fused polycyclic aromatic compounds.

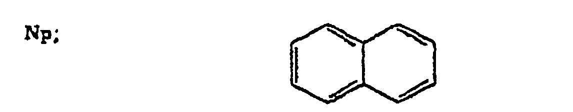

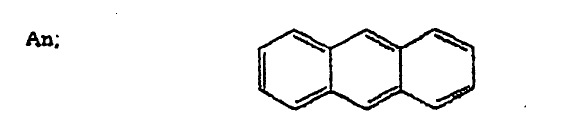

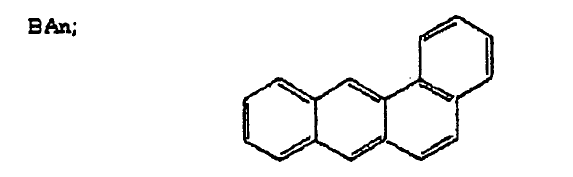

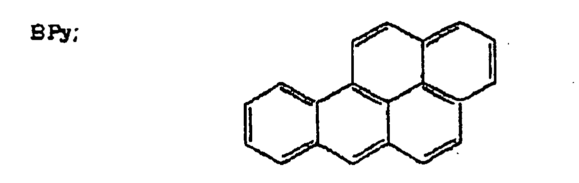

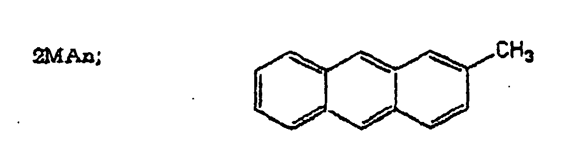

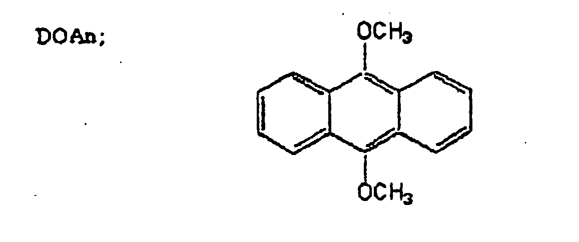

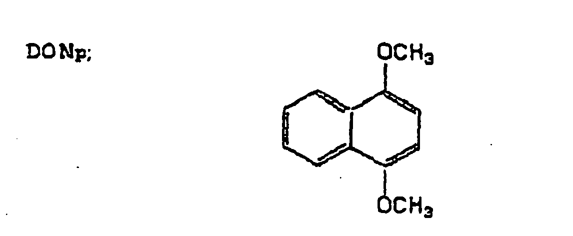

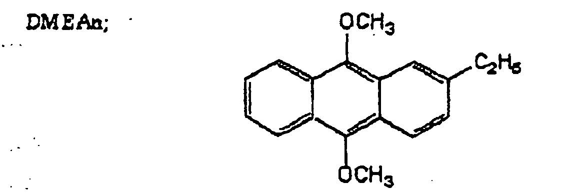

### (9) Other compounds: numerals in [ ] represent maximum absorption wavelengths.

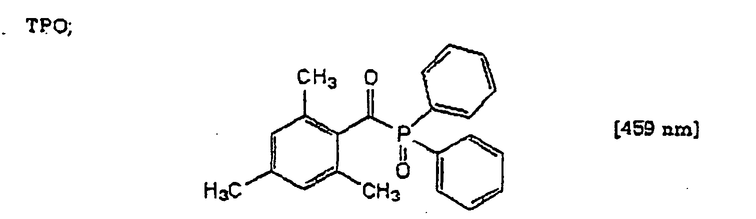

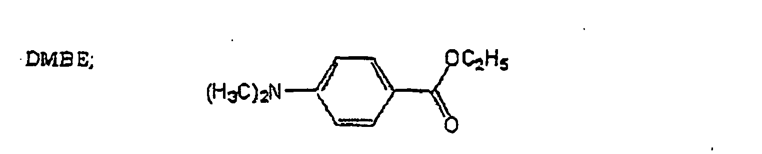

Properties of the materials appearing in the specification and in Examples were evaluated by the methods described below.

### (1) Gelling time.

A cationically polymerizable monomer solution containing a photopolymerization initiator was introduced, in amount (about 2.9 to 3.2 grams) forming a curing film having a thickness of 20 mm, into a sampling tube (volume; 6ml, inner diameter; 1.6 cm). Then, by using a dental light irradiator (TOKUSO POWER LITE, manufactured by Tokuyama Co.), light was irradiated from an irradiation distance of 0.5 cm (distance between the irradiating position and the surface of the solution). Here, the time when the monomer exhibited no fluidity was regarded to be a gelling time.

### (2) Curing property.

2.8 Grams of the solution was introduced into the above 6-ml sampling tube and was irradiated with light for 2 minutes in the same manner as the above method to evaluate in five steps depending upon the hardness of the cured material and the presence of unpolymerized portions. Namely, the samples having a sufficiently large hardness without at all containing uncured portion (liquid, jelly-like or rubber-like portion) were denoted by ⊚, the samples containing uncured portion but were partly cured to a depth of 15 mm were denoted by O, the samples that were cured to depths of not smaller than 10 mm but not larger than 15 mm were denoted'by ●, the samples that were partly cured but were not cured to a depth of 10 mm were denoted by Δ, and the samples that were gelled but did not acquire a sufficiently large hardness or were not at all cured were denoted by ×.

### (3) Cured depth.

3.2 Grams of the solution was introduced into the above 6-ml sampling tube and was irradiated with light for 2 minutes in the same manner as the above method. After the irradiation with light, the sampling tube was broken to take out the cured material which was, then, measured for its depth of curing at the shallowest portion to regard it as the cured depth.

### (4) Bending strength/flexural modulus of elasticity.

The polymerizable composition was filled in a metal mold measuring 2 x 2 x 25 mm and was cured by the irradiation with light for 1.5 minutes by using the light irradiator. The cured body was preserved at 37° C overnight. By using an Autograph (manufactured by Shimazu Corp.), five cured bodies in each group were measured for their three-point bending strengths and flexural moduli of elasticity maintaining a distance between fulcrums of 20 mm and a crosshead speed of 0.5 mm/min, and their average:values were calculated.

### (5) Volumetric shrinkage by polymerization.

A plunger made of SUS having a diameter of 3 mm and a height of 4 mm was introduced into a split mold made of SUS having a hole of a diameter of 3 mm and a height of 7 mm, such that the height of the hole was 3 mm. A curable composition was filled therein, and a polypropylene film was brought into pressed contact therewith from the upper side. The composition was placed on a glass plate equipped with a dental irradiator with the film surface faced downward and, then, a probe capable of measuring fine motion of the needle was brought into contact therewith from above the SUS plunger. The composition was cured by polymerization by using the dental light irradiator, and a shrinkage [%] 10 minutes after the start of light irradiation was calculated from the distance of motion of the probe in the up-and-down direction.

### (6) Unpolymerized surface.

The curable composition was placed in a state of being contacted to the air, and was irradiated with light for one minute using the dental light irradiator. The surfaces of the cured bodies that were sticky due to being not polymerized were denoted by X, and the surfaces without stickiness were denoted by O.

### (Examples 1 to 37)

To 100 parts by weight of the polymerizable monomers, there were added 0.2 parts by mass of photo acid-generating compounds, 0.05 parts by mass of photo radical-generating compounds and 0.05 parts by mass of fused polycyclic aromatic compounds as shown in Table 1 or 2, which were then dissolved in a dark place. The gelling times, curing properties and the cured depths of the solutions were as shown in Tables 1 and 2. In Examples 1 to 37, the cationically polymerizable monomers were used as the polymerizable monomers. In all Examples, the solutions were quickly gelled exhibiting favorable curing properties.

### (Examples 37 to 40)

To 100 parts by weight of the polymerizable monomers, there were added 0.2 parts by mass of photo acid-generating compounds, 0.05 parts by mass of photo radical-generating compounds and 0.05 parts by mass of fused polycyclic aromatic compounds as shown in Table 2, which were then dissolved in a dark place. The gelling times, curing properties and the cured depths of the solutions were as shown in Table 2. In Examples 38 to 41, Bis-GMA and 3G which were the radically polymerizable monomers were mixed to the cationically polymerizable monomers. In all Examples 38 to 41, the solutions were quickly gelled exhibiting favorable curing properties. Besides, the gelling times were very shorter than those of Examples 1 to 37.

### (Comparative Example 1)

To 100 parts by weight of OX-1, there were added 0.05 parts by mass of a photo acid-generating compound and 0-05 parts by mass of a fused polycyclic aromatic compound as shown in Table 3, which were then dissolved in a dark place in the same manner as in Examples 1 to 41. The gelling time, curing property and the cured depth of the solution were as shown in Table 3. Comparative Example 1 did not employ the photo acid-generating compound (A) which is the essential component of the present invention, and no curing behavior was exhibited at all.

### (Comparative Examples 2 to 16)

To 100 parts by weight of OX-1, there were added 0.2 parts by mass of the photo acid-generating compounds and 0.05 parts by mass of the fused polycyclic aromatic compounds as shown in Table 3, which were then dissolved in a dark place in the same manner as in Examples 1 to 41. The gelling times, curing properties and the cured depths of the solutions were as shown in Table 3. Comparative Examples 2 to 16 did not employ the photo radical-generating compound (B) which is the essential component of the present invention. The solutions had to be irradiated with light for longer periods of time until they were gelled than those of Examples. The cured depths were shallow.

### (Comparative Examples 17 to 24)

To 100 parts by weight of OX-1, there were added 0.2 parts by mass of the photo acid-generating compounds and 0.05 parts by mass of the photo radical-generating compounds as shown in Table 3, which were then dissolved in a dark place in the same manner as in Examples 1 to 41. The gelling times, curing properties and the cured depths of the solutions were as shown in Table 3. Comparative Examples 17 to 24 did not employ the fused polycyclic aromatic compound (C) which is the essential component of the present invention. In all of Comparative Examples 17 to 24, the solutions were not gelled or were gelled after irradiated with light for long periods of time. The cured depths were not sufficient, either.

### (Comparative Examples 25 to 28)

To 100 parts by weight of OX-1, there were added 0.2 parts by mass of the photo acid-generating compounds, 0.05 parts by mass of the photo radical-generating compounds and 0.05 parts by mass of the fused polycyclic aromatic compounds as shown in Table 4, which were then dissolved in a dark place in the same manner as in Examples 1 to 41. The gelling times, curing properties and the cured depths of the solutions were as shown in Table 4. Comparative Examples 25 to 28 employed fused polycyclic aromatic compounds without substituent instead of using the fused polycyclic aromatic compound (C) which is the essential component of the present invention. In all of Comparative Examples 25 to 28, the solutions had to be irradiated with light for extended periods of time before they were gelled. Cured depths were not sufficient, either.

### (Comparative Example 29)

To 100 parts by weight of OX-1, there were added 0.2 parts by mass of a photo acid-generating compound, 0.05 parts by mass of a photo radical-generating compound and 0.05 parts by mass of a fused polycyclic aromatic compound as shown in Table 4, which were then dissolved in a dark place in the same manner as in Examples 1 to 41. The gelling time, curing property and the cured depth of the solution were as shown in Table 4. Comparative Example 29 employed a fused polycyclic aromatic compound in which a methyl group was substituted for a carbon atom that was not adjacent the ring-fusion carbon atom instead of using the fused polycyclic aromatic compound (C) which is the essential component of the present invention. The solution had to be irradiated with light for extended periods of time before it was gelled. Cured depth was not sufficient, either.

### (Comparative Examples 30 to 33)

To 100 parts by weight of OX-1, there were added 0.2 parts by mass of the photo acid-generating compounds, 0.05 parts by mass of the photo radical-generating compounds and 0.05 parts by mass of the fused polycyclic aromatic compounds as shown in Table 4, which were then dissolved in a dark place in the same manner as in Examples 1 to 41. The gelling times, curing properties and the cured depths of the solutions were as shown in Table 4. Comparative Examples 30 to 33 employed fused polycyclic aromatic compounds for which a methoxy group only was substituted instead of using the fused polycyclic aromatic compound (C) which is the essential component of the present invention. In all of Comparative Examples 30 to 33, the solutions had to be irradiated with light for extended periods of time before they were gelled. Cured depths were not sufficient, either.

### (Comparative Examples 34 and 35)

To 100 parts by weight of OX-1, there were added 0.2 parts by mass of the photo acid-generating compounds, 0.05 parts by mass of the photo radical-generating compounds and 0.05 parts by mass of the fused polycyclic aromatic compounds as shown in Table 4, which were then dissolved in a dark place in the same manner as in Examples 1 to 41. The gelling times, curing properties and the cured depths of the solutions were as shown in Table 4. Comparative Examples 34 and 35 employed fused polycyclic aromatic compounds having an ethyl group substituted for the carbon atom that was not adjacent the ring-fusion carbon atom and further having a methoxy group as a substituent instead of using the fused polycyclic aromatic compound (C) which is the essential component of the present invention. In these Comparative Examples 34 and 35, the solutions had to be irradiated with light for extended periods of time before they were gelled. Cured depths were not sufficient, either.

### (Comparative Example 36)

To 100 parts by weight of OX-1, there were added 0.2 parts by mass of a photo acid-generating compound, 0.05 parts by mass of a photo radical-generating compound and 0.05 parts by mass of toluene as shown in Table 4, which were then dissolved in a dark place in the same manner as in Examples 1 to 41. The gelling time, curing property and the cured depth of the solution were as shown in Table 4. Comparative Example 36 employed toluene which is a non-fused compound instead of using the fused polycyclic aromatic compound (C) which is the essential component of the present invention. The solution had to be irradiated with light for extended periods of time before it was gelled. Cured depth was not sufficient, either.

### (Comparative Example 37)

To 100 parts by weight of OX-1, there were added 0.2 parts by mass of a photo acid-generating compound, 0.05 parts by mass of a photo radical-generating compound and 0.05 parts by mass of a fused polycyclic aromatic compound as shown in Table 4, which were then dissolved in a dark place in the same manner as in Examples 1 to 41. The gelling time, curing property and the cured depth of the solution were as shown in Table 4. Comparative Example 37 employed a photo radical-generating agent which is not of the photo acid-type instead of using the photo oxidation radical-generating compound (B) which is the essential component of the present invention. The solution had to be irradiated with light for extended periods of time before it was gelled. Cured depth was not sufficient, either.

### (Comparative Example 38)

To 100 parts by weight of OX-1, there were added 0.2 parts by mass of a photo acid-generating compound, 0.05 parts by mass of a photo radical-generating compound and 0.05 parts by mass of DMBE as shown in Table 4, which were then dissolved in a dark place in the same manner as in Examples 1 to 41. The gelling time, curing property and the-cured depth of the solution were as shown in Table 4. Comparative Example 38 employed the DMBE which is an electron-donating compound instead of using the fused polycyclic aromatic compound (C) which is the essential component of the present invention. The solution had to be irradiated with light for extended periods of time before it was gelled. Cured depth was not sufficient, either.

**Table 1**

| Example No. | Polymerizable monomer | Photo acid generator | Photo radical generator | Fused polycyclic aromatic compound | Gelling time | Curing property | Cured depth |
|---|---|---|---|---|---|---|---|
| 1 | OX-1 | DPISb | AnQ | DMAn | 21 | ⊚ | 20mm or more |
| 2 | OX-1 | DPIPB | AnQ | DMAN | 20 | ⊚ | 20mm or more |
| 3 | OX-1 | MDPISb | AnQ | DMAn | 19 | ⊚ | 20mm or more |
| 4 | OX-1 | BDPISb | AnQ | DMAn | 20 | ⊚ | 20nm or more |
| 5 | OX-1 | DMPSb | AnQ | DMAn | 33 | ⊚ | 20mm or more |
| 6 | OX-1 | DPISb | BAnQ | DMAn | 17 | ⊚ | 20mm or more |
| 7 | OX-1 | DPISb | FIN | DMAn | 23 | ⊚ | 20mm or more |
| 8 | OX-1 | DPISb | TXTN | DMAn | 22 | ⊚ | 20mm or more |
| 9 | OX-1 | DPISb | XTN | DMAn | 24 | ⊚ | 20mm or more |
| 10 | OX-1 | DPISb | CQ | DMAn | 16 | ⊚ | 20mm or more |
| 11 | OX-1 | DPISb | BZ | DMAn | 18 | ⊚ | 20mm or more |
| 12 | OX-1 | DPISb | KC | DMAn | 19 | ⊚ | 20mm or more |
| 13 | OX-1 | DPISb | BAnQ | DMNp | 22 | ⊚ | 20mm or more |
| 14 | OX-1 | DPISb | BAnQ | 9MAn | 18 | ⊚ | 20mm or more |
| 15 | OX-1 | DPISb | BAnQ | 9EAn | 18 | ⊚ | 20mm or more |
| 16 | OX-1 | DPISb | BAnQ | MOAn | 16 | ⊚ | 20mm or more |
| 17 | OX-1 | DPISb | BAnQ | DMOAn | 16 | ⊚ | 20mm or more |
| 18 | OX-1 | DPISb | BAnQ | DMMAn | 20 | ⊚ | 20mm or more |
| 19 | OX-1 | DPISb | BAnQ | PMAn | 16 | ⊚ | 20mm or more |
| 20 | OX-1 | DPISb | BAnQ | AAn | 15 | ⊚ | 20mm or more |
| 21 | OX-1 | DPISb | BAnQ | AnM | 15 | ⊚ | 20mm or more |
| 22 | OX-1 | DPISb | BAnQ | AnDM | 18 | ⊚ | 20mm or more |
| 23 | OX-1 | DPISb | BAnQ | OMAn | 18 | ⊚ | 20mm or more |
| 24 | OX-1 | DPISb | BAnQ | DMBAn | 13 | ⊚ | 20mm or more |
| 25 | OX-1 | DPISb | BAnQ | Chol | 14 | ⊚ | 20mm or more |
| 26 | OX-1 | DPISb | BAnQ | MBPy | 13 | ⊚ | 20mm or more |

**Table 2**

| Example No. | Polymerizable monomer | Photo acid generator | Photo radical generator | Fused polycyclic aromatic compound | Gelling time | Curing property | Cured depth |
|---|---|---|---|---|---|---|---|
| 27 | OX-1 | DPISb | BAnQ | DMBAn | 16 | ⊚ | 20mm or more |
| 28 | EP-1 | DPISb | BAnQ | DMBAn | 13 | ⊚ | 20mm or more |
| 29 | EP-2 | DPISb | BAnQ | DMBAn | 11 | ⊚ | 20mm or more |
| 30 | DV | DPISb | BAnQ | DMBAn | 4 | ⊚ | 20mm or more |
| 31 | OX-1;EP-1=95:5 | DPISb | BAnQ | DMBAn | 7 | ⊚ | 20mm or more |
| | (wt. ratio) | | | | | | |
| 32 | OX-2:EP-1=95:5 | DPISb | BAnQ | DMBAn | 9 | ⊚ | 20mm or more |
| 33 | OX-1:EP-2=95:5 | DPISb | BAnQ | DMBAn | 5 | ⊚ | 20mm or more |
| 34 | OX-2:EP-2=95:5 | DPISb | BAnQ | DMBAn | 5 | ⊚ | 20mm or more |
| 35 | OX-1:DV=95:5 | DPISb | BAnQ | DMBAn | 6 | ⊚ | 20mm or more |
| 36 | OX-2:DV=95:5 | DPISb | BAnQ | DMBAn | 9 | ⊚ | 20mm or more |
| 37 | BOE: EP-2=50 : 50 | DPISb | BAnQ | DMBAn | 12 | ⊚ | 20mm or more |
| 38 | OX-1:EP-2:Bis-GMA:3G =45.5:2.5:35:15 | DPISb | BAnQ | DMBAn | 3 | ⊚ | 20mm or more |
| 39 | OX-2:EP-2:Bis-GMA:3G =45.5:2.5:35:15 | DPISb | BAnQ | DMBAn | 3 | ⊚ | 20mm or more |
| 40 | OX-1:DV:Bis-GMA:3G =45.5:2.5:35:15 | DPISb | BAnQ | DMBAn | 3 | ⊚ | 20mm or more |
| 41 | OX-2:DV:Bis-GMA:3G =45.5:2.5:35:15 | DPISb | BAnQ | DMBAn | 3 | ⊚ | 20mm or more |

**Table 3**

| Comp. EX. No. | Polymerizable monomer | Photo acid generator | Photo radical generator | Fused polycyclic aromatic compound | Gelling time | Curing property | Cured depth |
|---|---|---|---|---|---|---|---|
| 1 | OX-1 | --- | BAnQ | DMBAn | not gelled | × | could not be measured |
| 2 | OX-1 | DPISb | -- | DMAn | 58 | ● | 11.2 mm |
| 3 | OX-1 | DPISb | -- | 9MAn | 62 | ● | 11.3 mm |
| 4 | OX-1 | DPISb | -- | 9EAn | 63 | ● | 12.0 mm |
| 5 | OX-1 | DPISb | -- | MOAn | 60 | ● | 12.1 mm |
| 6 | OX-1 | DPISb | -- | DMOAn | 56 | ● | 11.8 mm |
| 7 | OX-1 | DPISb | -- | DMMAn | 59 | ● | 12.0 mm |
| 9 | OX-1 | DPISb | -- | PMAn | 57 | ● | 10.9 mm |
| 10 | OX-1 | DPISb | -- | AAn | 57 | ● | 11.5 mm |
| 11 | OX-1 | DPISb | -- | AnM | 54 | ● | 12.8 mm |
| 12 | OX-1 | DPISb | -- | AnDM | 52 | ● | 12.3 mm |
| 13 | OX-1 | DPISb | -- | CMAn | 59 | ● | 11.2 mm |
| 14 | OX-1 | DPISb | -- | DMBAn | 50 | ● | 13.4 mm |
| 15 | OX-1 | DPISb | -- | Chl | 48 | ● | 13.2 mm |
| 16 | OX-1 | DPISb | -- | MBPy | 43 | ● | 12.2 mm |
| 17 | OX-1 | DPISb | AnQ | -- | 65 | △ | 3.3 mm |
| 18 | OX-1 | DPISb | BAnQ | -- | 40 | △ | 7.2 mm |
| 19 | OX-1 | DPISb | FLN | -- | not gelled | × | could not be measured |
| 20 | OX-1 | DPISb | TXTN | -- | 39 | △ | 9 mm |
| 21 | OX-1 | DPISb | XTN | -- | 70 | × | could not be measured |
| 22 | OX-1 | DPISb | CQ | -- | 45 | △ | 6.3 mm |
| 23 | OX-1 | DPISb | BZ | -- | 47 | △ | 6.2 mm |
| 24 | OX-1 | DPISb | KC | -- | 43 | △ | 4.2 mm |

**Table 4**

| Comp. EX. No. | Polymerizable monomer | Photo acid generator | Photo radical generator | Fused polycyclic aromatic compound and others | Gelling time | Curing property | Cured depth |
|---|---|---|---|---|---|---|---|
| 25 | OX-1 | DPISb | BAnQ | Np | 41 | △ | 7.2 mm |
| 26 | OX-1 | DPISb | BAnQ | An | 39 | △ | 7.3 mm |
| 27 | OX-1 | DPISb | BAnQ | BAn | 35 | △ | 7.5 mm |
| 28 | OX-1 | DPISb | BAnQ | BPy | 32 | △ | 7.0 mm |
| 29 | OX-1 | DPISb | BAnQ | 2MAn | 37 | △ | 9.1 mm |
| 30 | OX-1 | DPISb | BAnQ | DOAn | 39 | △ | 9.2 mm |
| 31 | OX-1 | DPISb | TXTN | DOAn | 38 | △ | 9.5 mm |
| 32 | OX-1 | DPISb | BAnQ | DONp | 41 | △ | 6.2 mm |
| 33 | OX-1 | DPISb | TXTN | DONp | 39 | △ | 7.8 mm |
| 34 | OX-1 | DPISb | BAnQ | DMEAn | 32 | ● | 12.1 mm |
| 35 | OX-1 | DPISb | TXTN | DMEAn | 33 | △ | 7.2 mm |
| 36 | OX-1 | DPISb | BAnQ | toluene | 42 | △ | 7.0 mm |
| 37 | OX-1 | DPISb | TPO | DMBAn | 52 | △ | 6.2 mm |
| 3B | OX-1 | DPISb | CQ | DMBE | 38 | △ | 9.3 mm |

### (Examples 42 to 52)

The following solutions A to G were prepared.

### Solution A:

To 100 parts by mass of a mixture of monomers of a weight ratio of OX-2/EP-1 = 95/5, there were dissolved 0.8 parts by mass of DPISb, 0.1 part by mass of BAnQ and 0.1 part by mass of DMAn in a dark place to prepare a solution A.

### Solution B:

By using a mixture of monomers of OX-2/EP-2 = 95/5, there was prepared a solution B in the same manner as the solution A.

### Solution C:

By using a mixture of monomers of OX-2/DV = 95/5, there was prepared a solution C in the same manner as the solution A.

### Solution D:

By using a mixture of monomers of BOE/EP-2 =50/50, there was prepared a solution D in the same manner as the solution A.

### Solution E:

By using a mixture of monomers of OX-1:EP-2:Bis-GMA:3G = 45.5:2.5:35:15, there was prepared a solution E in the same manner as the solution A.

### Solution F:

By using a mixture of monomers of OX-2:EP-2:Bis-GMA:3G = 45.5:2.5:35:15, there was prepared a solution F in the same manner as the solution A.

### Solution G:

By using a mixture of monomers of Bis-GMA:3G = 70:30, there was prepared a solution G in the same manner as the solution A.

20 Grams of a quartz powder (particle size of 5 µm) was suspended in 80 ml of an acetic acid aqueous solution of which the pH was adjusted to 4.0, and to which was added dropwise 0.8 g of 3-glycidyloxypropyltrimethoxysilane with stirring. After stirred for one hour, water was distilled off by using an evaporator, the obtained solid material was milled in a mortar and was dried under a reduced pressure at 80⁻C for 15 hours. After drying, the obtained powder was labeled as an inorganic filler F1.

Similarly, there were prepared an inorganic filler F2 from spherical silica (particle size of 0.2 to 2 µm), an inorganic filler F3 from spherical silica-zirconia (particle size of 0.4 µm), an inorganic filler F4 from spherical silica-zirconia (particle size of 0.2 µm), an inorganic filler F5 from spherical silica-titania (particle size of 0.1 µm), and an inorganic filler F6 from milled silica-zirconia (particle size of 5 µm). By using 3-methacryloxypropyltrimethoxysilane instead of the 3-glycidyloxypropyltrimethoxysilane, further, there were prepared an inorganic filler F7 by conducting the same surface treatment for the spherical silica-zirconia (particle size of 0.2 µm) and an inorganic filler F8 by conducting the same surface treatment for the milled silica-zirconia (particle size of 5 µm).

The thus prepared various inorganic fillers and various solutions were mixed together in an agate mortar, and the mixture was defoamed in vacuum to remove bubbles thereby to obtain polymerizable compositions. Further, the filler contents for the polymerizable compositions were expressed by weight ratios and were regarded to be filling ratios (%). Table 5 shows various polymerizable compositions, bending strengths, flexural module of elasticity, volumetric shrinkages due to.polymerization and unpolymerized surfaces.

**Table 5**

| Example No. | Filler wt ratio | Solution | Filling ratio % | Bending strength /MPa (standard deviation) | Flexural module of elasticity/ GPa (standard deviation) | Volumetric shrinkage % | Unpolymerized surface |
|---|---|---|---|---|---|---|---|
| 42 | F1 : F2 = 60 : 40 | A | 86 | 152.3(5.2) | 12.3(0.81) | 0.83 | ○ |
| 43 | F1 : F2 = 60 : 40 | B | 86 | 147.2(4.5) | 11.7(0.83) | 0.85 | ○ |
| 44 | F1 : F2 = 60 : 40 | C | 86 | 145.7(3.2) | 11.5(0.70) | 0.86 | ○ |
| 45 | F1 : F2 = 60 : 40 | D | 86 | 120.7(3.4) | 9.5(0.72) | 0.51 | ○ |
| 46 | F3 : F5 = 70 : 30 | A | 82 | 152.8(3.4) | 10.3(0.68) | 0.91 | ○ |
| 47 | F4 : F6 = 40 : 60 | A | 83 | 138.3(4.0) | 9.8(0.71) | 0.88 | ○ |
| 48 | F1 : F2 = 60 : 40 | E | 86 | 163.3(5.8) | 13.6(0.79) | 1.01 | ○ |
| 49 | F1 : F2 = 60 : 40 | F | 86 | 161.5(5.4) | 13.5(0.76) | 1.02 | ○ |
| 50 | F3 : F5 = 70 : 30 | E | 82 | 162.8(4.0) | 13.6(0.63) | 1.08 | ○ |
| 51 | F4 : F6 = 40 : 60 | E | 83 | 158.2(3.5) | 12.9(0.71) | 1.07 | ○ |
| 52 | F7 : F8 = 40 : 60 | G | 83 | 160.3(6.3) | 10.2(0.51) | 1.31 | × |

The photopolymerization initiator of the present invention possesses sufficient sensitivity even for the wavelengths in the visible light region, and makes it possible to obtain an increased depth of curing by the irradiation with light in a short period of time even by using a visible light irradiator that has been placed in dental use. Further, the polymerized and cured material thereof exhibits a variety of excellent mechanical properties.

It is further possible to polymerize and cure a cationically polymerizable monomer that is not impaired for its polymerization by oxygen. By using the photopolymerizable composition comprising the photopolymerization initiator and the cationically polymerizable monomer of the present invention, therefore, it is made possible to obtain a cured material without unpolymerized layer in the surface even without using any particular means for blocking oxygen. Even when the photopolymerizable composition is polymerized and cured in a place where it is difficult to block oxygen like in an oral cavity, therefore, there are easily obtained favorable properties even without polishing the surfaces.

In general, further, the cationically polymerizable monomer shrinks less when it is polymerized than the radically polymerizable monomers, and blending the filler further decreases the shrinking by polymerization. By using the polymerizable composition as a composite resin for dental use, therefore, excellent sealing is accomplished without developing a gap in the interface between the composite resin and the dentin at the time of polymerization, eliminating the probability of secondary decaying that is caused by the infiltration of bacteria through the gap after the therapy. Further, the photopolymerization initiator of the present invention is capable of effeciently polymerizing the radically polymerizable monomer, too, and makes it possible to firmly adhere a dental adhesive comprising chiefly the radically polymerizable monomer to a filler restorative comprising chiefly the cationically polymerizable monomer.

Further, the photopolymerizable composition containing the photopolymerization initiator of the present invention can be favorably used in the dental applications like, for example, a dental adhesive in addition to being used as a dental filler restorative as represented by the above-mentioned composite resin, and can, further, be used as various materials that utilize photopolymerization, such as photoresist material, plate material for printing, hologram material and the like materials.

## Claims

1. A photopolymerization initiator comprising (A) a photo acid-generating compound, (B) a photo oxidation radical-generating compound, and (c) a fused polycyclic aromatic compound having a fused aromatic ring and a molecular structure in which at least one carbon atom adjacent to a ring-fusion carbon atom in the fused aromatic ring is bonded to a saturated carbon atom having at least one hydrogen atom.

2. A photopolymerization initiator according to claim 1, wherein the photo acid-generating compound (A) is a diaryliodonium salt compound.

3. A photopolymerization initiator according to claim 1, wherein the photo oxidation radical-generating compound (B) is at least the one selected from the group consisting of a diarylketone compound, an α-diketone compound and a ketocoumarin compound.

4. A photopolymerization initiator according to claim 1, wherein the fused polycyclic aromatic compound (C) is represented by the following general formula (1),
R¹R²CₛₐₜH-A-(R³)ₙ (1)
wherein n is an integer of 0 to 6,
A is an aromatic hydrocarbon group having a valency of (n + 1) and with which 2 to 6 benzene rings are fused,
Cₛₐₜ is a saturated carbon atom bonded to a carbon atom adjacent to the ring-fusion carbon atom of the aromatic hydrocarbon group,
R¹ and R² are, independently from each other, hydrogen atoms, halogen atoms, hydroxyl groups, mercapto groups or monovalent organic residues having 1 to 10 carbon atoms, and
R³ is a halogen atom, a hydroxyl group, a mercapto group or a monovalent organic residues having 1 to 10 carbon atoms, and when n is 2 or more, R³s may be different from each other, and
wherein any two groups selected from R¹, R² and R³ may be bonded together, or two R³ groups may be bonded together to form a non-aromatic ring.

5. A photopolymerization initiator according to claim 1, wherein the photo oxidation radical-generating compound (B) is contained in an amount of 0.001 to 20 mols per mol of the photo acid-generating compound (A).

6. A photopolymerization initiator according to claim 1, wherein the fused polycyclic aromatic compound (C) is contained in an amount of 0.0005 to 20 mols per mol of the photo acid-generating compound (A).

7. A photopolymerizable composition containing a photopolymerization initiator of claim 1 and a polymerizable monomer.

8. A photopolymerizable composition according to claim 7, wherein the photopolymerization initiator is blended in such an amount that the photo acid-generating compound (A) in said photopolymerization initiator is in an amount of 0.001 to 10 parts by mass per 100 parts by mass of the polymerizable monomer.

9. A photopolymerizable composition according to claim 7, wherein the polymerizable monomer is a cationically polymerizable monomer.

10. A dental material comprising a photopolymerizable composition of claim 7.

## Patentansprüche

1. Photopolymerisationsinitiator, umfassend (A) eine eine Photosäure erzeugende Verbindung, (B) eine ein Photooxidationsradikal erzeugende Verbindung und (C) eine kondensierte, polycyclische, aromatische Verbindung mit einem kondensierten aromatischen Ring und einer Molekülstruktur, in der mindestens ein Kohlenstoffatom in Nachbarschaft zu einem Ringkondensations-Kohlenstoffatom im kondensierten aromatischen Ring an ein gesättigtes Kohlenstoffatom mit mindestens einem Wasserstoffatom gebunden ist.

2. Photopolymerisationsinitiator nach Anspruch 1, wobei es sich bei der eine Photosäure erzeugenden Verbindung (A) um eine Diaryliodoniumsalz-Verbindung handelt.

3. Photopolymerisationsinitiator nach Anspruch 1, wobei es sich bei der ein Photooxidationsradikal erzeugenden Verbindung (B) um mindestens eine Verbindung handelt, die aus der Gruppe ausgewählt ist, die aus einer Diarylketonverbindung, einer α-Diketonverbindung und einer Ketocumarinverbindung ausgewählt ist.

4. Photopolymerisationsinitiator nach Anspruch 1, wobei die kondensierte, polycyclische, aromatische Verbindung (C) durch die folgende allgemeine Formel (1) wiedergegeben wird:
R¹R²CₛₐₜH-A-(R³)ₙ (1)
wobei n eine ganze Zahl mit einem Wert von 0 bis 6 bedeutet,
A eine aromatische Kohlenwasserstoffgruppe mit einer Wertigkeit von (n+1) bedeutet, mit der 2 bis 6 Benzolringe kondensiert sind,
Cₛₐₜ ein gesättigtes Kohlenstoffatom bedeutet, das an einem Kohlenstoffatom in Nachbarschaft zum Ringkondensations-Kohlenstoffatom der aromatischen Kohlenwasserstoffgruppe gebunden ist,
R¹ und R² jeweils unabhängig voneinander Wasserstoffatome, Halogenatome, Hydroxylgruppen, Mercaptogruppen oder einwertige organische Reste mit 1 bis 10 Kohlenstoffatomen bedeuten und
R³ ein Halogenatom, eine Hydroxylgruppe, eine Mercaptogruppe oder einen einwertigen organischen Rest mit 1 bis 10 Kohlenstoffatomen bedeutet, und, wenn n einen Wert von 2 oder mehr hat, die Reste R³ voneinander verschieden sein können, und
wobei beliebige zwei Gruppen, die aus R¹, R² und R³ ausgewählt sind, miteinander verbunden sein können oder zwei R³-Gruppen miteinander unter Bildung eines nicht-aromatischen Rings verbunden sein können.

5. Photopolymerisationsinitiator nach Anspruch 1, wobei die ein Photooxidationsradikal erzeugende Verbindung (B) in einer Menge von 0,001 bis 20 Mol pro Mol der eine Photosäure erzeugenden Verbindung (A) enthalten ist.

6. Photopolymerisationsinitiator nach Anspruch 1, wobei die kondensierte, polycyclische, aromatische Verbindung (C) in einer Menge von 0,0005 bis 20 Mol pro Mol der eine Photosäure erzeugenden Verbindung (A) enthalten ist.

7. Photopolymerisierbare Zusammensetzung, enthaltend einen Photopolymerisationsinitiator nach Anspruch 1 und ein polymerisierbares Monomeres.

8. Photopolymerisierbare Zusammensetzung nach Anspruch 7, wobei der Photopolymerisationsinitiator in einer solchen Menge zugemischt ist, dass die eine Photosäure erzeugende Verbindung (A) im Photopolymerisationsinitiator in einer Menge von 0,001 bis 10 Masseteilen pro 100 Masseteile des polymerisierbaren Monomeren vorliegt.

9. Photopolymerisierbare Zusammensetzung nach Anspruch 7, wobei es sich beim polymerisierbaren Monomeren um ein kationisch polymerisierbares Monomeres handelt.

10. Dentalmaterial, umfassend eine photopolymerisierbare Zusammensetzung nach Anspruch 7.

## Revendications

1. Initiateur de photopolymérisation comprenant (A) un composé produisant un photo-acide, (B) un composé produisant un radical de photo-oxydation et (C) un composé aromatique polycyclique condensé ayant un noyau aromatique condensé et une structure moléculaire dans laquelle au moins un atome de carbone adjacent à un atome de carbone de condensation de noyau dans le noyau aromatique condensé est lié à un atome de carbone saturé ayant au moins un atome d'hydrogène.

2. Initiateur de photopolymérisation selon la revendication 1, dans lequel le composé (A) produisant un photo-acide est un composé de sel de diaryliodonium.

3. Initiateur de photopolymérisation selon la revendication 1, dans lequel le composé (B) produisant un radical de photo-oxydation est au moins l'un choisi dans le groupe consistant en un composé diarylcétone, un composé α-dicétone et un composé cétocoumarine.

4. Initiateur de photopolymérisation selon la revendication 1, dans lequel le composé (C) aromatique polycyclique condensé est représenté par la formule générale (1) suivante,
R¹R²CₛₐₜH-A-(R³)ₙ (1)
dans laquelle n est un nombre entier de 0 à 6,
A est un groupe aromatique hydrocarboné ayant une valence de (n + 1) et avec lequel 2 à 6 noyaux benzène sont condensés,
Cₛₐₜ est un atome de carbone saturé lié à un atome de carbone adjacent à l'atome de carbone de condensation de noyau du groupe aromatique hydrocarboné,
R¹ et R² sont, indépendamment l'un de l'autre, des atomes d'hydrogène, des atomes d'halogène, des groupes hydroxyle, des groupes mercapto ou des résidus organiques monovalents ayant de 1 à 10 atomes de carbone, et
R³ est un atome d'halogène, un groupe hydroxyle, un groupe mercapto ou un résidu organique monovalent ayant de 1 à 10 atomes de carbone, et lorsque n vaut 2 ou plus, les R³ peuvent être différents les uns des autres, et
dans lesquels deux groupes quelconques choisis parmi R¹, R² et R³ peuvent être liés ensemble, ou deux groupes R³ peuvent être liés ensemble pour former un noyau non aromatique.

5. Initiateur de photopolymérisation selon la revendication 1, dans lequel le composé (B) produisant un radical de photo-oxydation est contenu en une quantité de 0,001 à 20 moles par mole du composé (A) produisant un photo-acide.

6. Initiateur de photopolymérisation selon la revendication 1, dans lequel le composé (C) aromatique polycyclique condensé est contenu en une quantité de 0,0005 à 20 moles par mole du composé (A) produisant un photo-acide.

7. Composition photopolymérisable contenant un initiateur de photopolymérisation selon la revendication 1 et un monomère polymérisable.

8. Composition photopolymérisable selon la revendication 7, dans laquelle l'initiateur de photopolymérisation est mélangé en une quantité telle que le composé (A) produisant un photo-acide dans ledit initiateur de photopolymérisation est en une quantité de 0,001 à 10 parties en poids pour 100 parties en poids du monomère polymérisable.

9. Composition photopolymérisable selon la revendication 7, dans laquelle le monomère polymérisable est un monomère cationiquement polymérisable.

10. Matériau dentaire comprenant une composition photopolymérisable selon la revendication 7.
